(19) Europäisches Patentamt
European Patent Office
Office européen des brevets

(11) EP 0 572 142 B1

(12) EUROPEAN PATENT SPECIFICATION

(45) Date of publication and mention of the grant of the patent: **31.07.1996 Bulletin 1996/31**

(51) Int. Cl.$^6$: **C07D 249/08**, A01N 43/82, C07D 401/12, C07F 7/10, A01N 55/00

(21) Application number: **93303700.4**

(22) Date of filing: **13.05.1993**

# (54) Novel 3,5-diphenyl substituted 1,2,4-triazoles and their use as insecticides and acaricides

Neue 3,5-Diphenyl-substituierte 1,2,4-Triazole und ihre Verwendung als Insektizide und Akarizide

Nouveaux 1,2,4-triazoles 3,5-diphényl-substitués et leur utilisation comme insecticides et acaricides

(84) Designated Contracting States:
**DE ES FR GB IT NL**

(30) Priority: **29.05.1992 JP 161759/92**

(43) Date of publication of application:
**01.12.1993 Bulletin 1993/48**

(73) Proprietors:
- **KUMIAI CHEMICAL INDUSTRY CO., LTD. Tokyo (JP)**
- **IHARA CHEMICAL INDUSTRY Co., Ltd. Taitoh-ku, Tokyo 110 (JP)**

(72) Inventors:
- **Ozaki, Masami, c/o K I CHEMICAL RES. INST. CO. LTD Iwata-gun, Shizuoka Pref. (JP)**
- **Ikeda, Atsuhiko, c/o K I CHEMICAL RES. INST.CO.LTD Iwata-gun, Shizuoka Pref. (JP)**
- **Honami, Reijiro, c/o K I CHEMICAL RES. INST.CO.LTD Iwata-gun, Shizuoka Pref. (JP)**
- **Yumita, Takashi, c/o K I CHEMICAL RES. INST.CO.LTD Iwata-gun, Shizuoka Pref. (JP)**
- **Yano, Hiroyuki Ogasa-gun, Shizuoka Pref. (JP)**
- **Hirano, Tadayoshi Kakegawa City, Shizuoka Pref. (JP)**
- **Minoguchi, Naokazu Ogasa-gun, Shizuoka Pref. (JP)**
- **Izawa, Norihiko Ogasa-gun, Shizuoka Pref. (JP)**

(74) Representative: **Ede, Eric**
**Fitzpatricks,**
**4 West Regent Street**
**Glasgow G2 1RS, Scotland (GB)**

(56) References cited:
**EP-A- 0 036 711** **EP-A- 0 185 256**

Remarks:
The file contains technical information submitted after the application was filed and not included in this specification

EP 0 572 142 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.0/3.4

## Description

This invention relates to novel triazole derivatives as well as insecticide and acaricide containing the same as an active ingredient.

Japanese Patent laid open No. 56-154464 and DE-A-363-1511 disclose that various triazole derivatives develop insecticidal and acaricidal activities. However, it can not be said that the insecticidal and acaricidal activities of these compounds described in these specifications are satisfactory.

An insecticidal compound is described in EP-A-0 036 711 (equivalent to JP-A-56-154464) which is characterized by bonding a phenyl group to the 5-position of 1H-1,2,4-triazole, but there is no description of the groups used in the invention to be more particularly described below (namely a 4-decylphenyl group, 4-phenyloxymethyl group, 4-phenyloxymethylphenyl group or the like). The compounds according to the present invention have improved insecticidal and acaricidal activities as compared with the compounds described in JP-A-56-154464.

EP-A-0 185 256 describes a compound characterized by bonding the o-trifluoromethylphenyl group or 4-trifluoromethyl-3-pyridyl group to the 5-position of 1H-1,2,4-triazole, which is different from the invention to be described hereinbelow (which has a 4-decylphenyl group, 4-phenyloxymethyl group, 4-phenyloxymethylphenyl group or the like). EP-A-0 185 256 discloses that such a compound is useful as an insecticide and acaricide.

Up to the present, various compounds such as organophosphorus compound, organotin compound and the like have been used for the control of pests in farm and garden crops and mites. However, these compounds have been used over many years, so that the above injurious insects have a resistance to chemicals to a certain extent and it recently becomes difficult to control these insects. Particularly, this tendency is conspicuous in lepidopteran injurious insects, mites and aphids and becomes serious. As a result, it is demanded to develop new types of insecticide and acaricide having a different function.

The inventors have made various studies in order to create novel insecticides and acaricides having a very high effect against wide injurious pests and capable of safely using, which have never been found in the conventional technique, in the development of the insecticide and acaricide having a function different from that of the conventional ones.

Further, the inventors have synthesized various triazole derivatives and examined their physiological activities. As a result, the inventors have found that novel triazole derivatives having a general formula [I] as mentioned later have an excellent effect against wide injurious pests in farm and garden crops, particularly lepidopteran injurious insects, mites and aphids and also develop a very high effect against eggs and larvae of mites and larvae of aphids having a resistance to the conventional chemicals, and the invention has been accomplished.

According to the invention, there is the provision of a triazole derivative having the following general formula [I]:

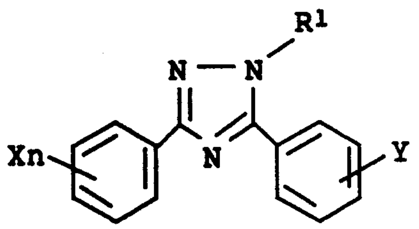

[ I ]

[wherein $R^1$ is an alkyl group, X is a hydrogen atom, a halogen atom, an alkyl group, an alkoxy group, an alkylthio group, a nitro group, a cyano group or a trifluoromethyl group, n is an integer of 1-5 provided that when n is 2 or more, X may be an optional combination of same or different atoms or groups, and Y is an alkenyl group, an alkynyl group, an alkoxyalkyl group, an alkoxyalkoxy group, an alkylthioalkyl group, a cycloalkyl group, a cycloalkylalkoxy group, a cycloalkylalkyl group, a cycloalkylalkenyl group, a cycloalkylalkynyl group, a trialkylsilylalkyl group, a trialkylsilylalkoxy group, an alkyl group having a carbon number of not less than 7, an alkoxy group having a carbon number of not less than 7, an alkylthio group having a carbon number of not less than 7, an alkylsulfinyl group having a carbon number of not less than 7, an alkylsulfonyl group having a carbon number of not less than 7 or a group represented by the following general formula (1):

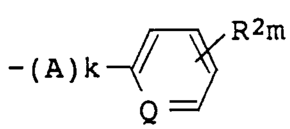

(wherein A is an oxygen atom, a sulfur atom, a lower alkylene group, a lower alkyleneoxy group, an oxy-lower alkylene group or a lower alkyleneoxyalkylene group, k is 0 or 1, Q is CH- group or a nitrogen atom, $R^2$ is a hydrogen atom, a halogen atom, an alkyl group, an alkoxy group, trifluoromethyl group or trifluoromethoxy group, m is an integer of 1-5 provided that when m is 2 or more, $R^2$ may be an optional combination of same or different atoms or groups)].

Furthermore, the invention provides an insecticide or an acaricide containing the above triazole derivative as an active ingredient.

Throughout the specification, the term "lower" means that the carbon number in the group added with this term is not more than 6.

Further, the term "alkyl group" means a straight or branched-chain alkyl group having a carbon number of 1-30, which includes, for example, methyl group, ethyl group, n-propyl group, isopropyl group, n-butyl group, isobutyl group, sec-butyl group, t-butyl group, n-pentyl group, isoamyl group, neopentyl group, n-hexyl group, isohexyl group, 3,3-dimethylbutyl group, n-heptyl group, 5-methylhexyl group, 4-methylhexyl group, 3-methylhexyl group, 4,4-dimethylpentyl group, n-octyl group, 6-methylheptyl group, n-nonyl group, 7-methyloctyl group, n-decyl group, 8-methylnonyl group, n-undecyl group, 9-methyldecyl group, n-dodecyl group, 10-methylundecyl group, n-tridecyl group, 11-methyldodecyl group, n-tetradecyl group, 12-methyltridecyl group, n-pentadecyl group, 13-methyl-tetradecyl group, n-hexadecyl group, n-heptadecyl group, n-octadecyl group, n-nonadecyl group, n-eicosyl group and the like.

The terms "alkoxy group", "alkylthio group", "alkylsulfinyl group" and "alkylsulfonyl group" are (alkyl)-O- group, (alkyl)-S- group, (alkyl)-SO- group, and (alkyl)-$SO_2$ group in which the alkyl portion has the same meaning as mentioned above, respectively.

The term "halogen atom" means fluorine, chlorine, bromine and iodine.

The term "alkenyl group" means a straight or branched-chain alkenyl group having a carbon number of 2-20, which includes, for example, vinyl group, propenyl group, isopropenyl group, butenyl group, pentenyl group, hexenyl group, heptenyl group, octenyl group, 3-methyl-1-butenyl group, 4-methyl-1-pentenyl group and the like.

The term "alkynyl group" means a straight or branched-chain alkynyl group having a carbon number of 2-20, which includes, for example, ethynyl group, propynyl group, butynyl group, pentynyl group, hexynyl group, 3,3-dimethyl-1-butynyl group, 4-methyl-1-pentynyl group, 3-methyl-1-pentynyl group, 5-methyl-1-hexynyl group, 4-methyl-1-hexynyl group, 3-methyl-1-hexynyl group, heptynyl group, octynyl group, nonynyl group, decynyl group, undecynyl group, dodecynyl group, tridecynyl group, tetradecynyl group, pentadecynyl group, hexadecynyl group and the like.

The term "cycloalkyl group" means a cycloalkyl group having a carbon number of 3-12, which includes, for example, cyclopropyl group, cyclobutyl group, cyclopentyl group, cyclohexyl group, cycloheptyl group, cyclooctyl group and the like.

The term "cycloalkylalkyl group" means a cycloalkylalkyl group having a carbon number of 6-12, which includes, for example, cyclopentylmethyl group, cyclohexylmethyl group, cyclopentylethyl group, cyclohexylethyl group, cyclopentylpropyl group, cyclohexylpropyl group, cyclohexylpentyl group and the like.

The term "cycloalkylalkoxy group" means a (cycloalkylalkyl)-O- group in which the cycloalkylalkyl portion has the same meaning as mentioned above.

The term "cycloalkylalkenyl group" means a cycloalkylalkenyl group having a carbon number of 5-12, which includes, for example, cyclopentylvinyl group, cyclohexylvinyl group, 3-cyclopentyl-1-propenyl group, 3-cyclohexyl-1-propenyl group, 5-cyclohexyl-1-pentenyl group and the like.

The term "cycloalkylalkynyl group" means a cycloalkylalkynyl group having a carbon number of 5-12, which includes, for example, cyclopentylethynyl group, cyclohexylethynyl group, 3-cyclopentyl-1-propynyl group, 3-cyclohexyl-1-propynyl group and the like.

The term "tri(lower alkyl)silyl lower alkyl group" includes, for example, trimethylsilylmethyl group, dimethylethylsilylmethyl group, butyldimethylsilylmethyl group and the like.

The term "tri(lower alkyl)silyl lower alkoxy group" means [tri(lower alkyl)silyl lower alkyl]-O- group in which the tri(lower alkyl)silyl lower alkyl portion has the same meaning as mentioned above.

The term "lower alkylene group" means a straight or branched-chain alkylene group having a carbon number of 1-4, which includes, for example, $-CH_2-$, $-CH_2CH_2-$, $-CH(CH_3)-$, $-CH_2CH_2CH_2-$, $-CH(CH_3)CH_2-$, $-C(CH_3)_2-$, $-CH_2CH_2CH_2CH_2-$, $-CH(CH_3)CH_2CH_2-$, $-CH_2CH(CH_3)CH_2-$ and the like.

The term "lower alkyleneoxy group" means -(lower alkylene)-O- group in which the lower alkylene portion has the same meaning as mentioned above.

The term "oxy-lower alkylene group" means -O-(lower alkylene)- group in which the lower alkylene portion has the same meaning as mentioned above.

The term "lower alkyleneoxyalkylene group" means -(lower alkylene)-O-(lower alkylene)- group in which the lower alkylene portion has the same meaning as mentioned above.

As a preferable compound according to the invention, there are mentioned compounds having the general formula [I] wherein $R^1$ is a straight or branched-chain alkyl group having a carbon number of 1-6, preferably methyl group, X is a hydrogen atom, a halogen atom, a straight or branched-chain alkyl group having a carbon number of 1-4, a nitro group, a cyano group or trifluoromethyl group, n is an integer of 1-3 provided that when n is 2 or 3, X may be an optional

combination of same or different atoms or groups, Y is a straight or branched-chain alkyl group having a carbon number of 7-20, a cycloalkyl group having a carbon number of 3-12, a cycloalkylalkyl group having a carbon number of 6-12, a straight or branched-chain alkoxy group having a carbon number of 7-16, a cycloalkylalkoxy group having a carbon number of 7-12, a straight or branched-chain alkylthio group having a carbon number of 7-16, an alkylsulfinyl group, an alkylsulfonyl group, a straight or branched-chain alkenyl group having a carbon number of 3-16, a cycloalkylalkenyl group having a carbon number of 5-12, a straight or branched-chain alkynyl group having a carbon number of 3-16, a cycloalkylalkynyl group having a carbon number of 5-12, a tri(lower alkyl)silyl lower alkyl group, a tri(lower alkyl)silyl lower alkoxy group or a group represented by the formula (1) (wherein A is an oxygen atom, a sulfur atom, a lower alkylene group having a carbon number of 1-4, methyleneoxy group or oxymethylene group, k is 0 or 1, Q is CH- group or a nitrogen atom, $R^2$ is a hydrogen atom, a halogen atom, a lower alkyl group, a lower alkoxy group, trifluoromethyl group or trifluoromethoxy group, and m is an integer of 1-3 provided that when m is 2 or 3, $R^2$ may be an optional combination of same or different atoms or groups).

Concrete examples of the compounds having the general formula [I] according to the invention are shown in Tables 1 to 10. Moreover, the compound No. is referred in subsequent description.

Table 1

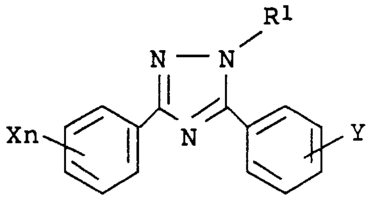

| Compound No. | $R^1$ | Xn | Y | Melting point (°C) or refractive index($n_D^{20}$) |
|---|---|---|---|---|
| 1 | $CH_3$ | H | $4-C_7H_{15}$ | 1.5819 |
| 2 | $CH_3$ | 2-F | $4-C_7H_{15}$ | 1.5650 |
| 3 | $CH_3$ | 2-Cl | $4-C_7H_{15}$ | 1.5816 |
| 4 | $CH_3$ | 2-Br | $4-C_7H_{15}$ | 1.5924 |
| 5 | $CH_3$ | 2-I | $4-C_7H_{15}$ | 1.6025 |
| 6 | $CH_3$ | $2,3,4,5,6-F_5$ | $4-C_7H_{15}$ | 1.5252 |
| 7 | $CH_3$ | $2-CH_3$ | $4-C_7H_{15}$ | 1.5803 |
| 8 | $CH_3$ | $2-OCH_3$ | $4-C_7H_{15}$ | 1.5840 |
| 9 | $CH_3$ | $2-SCH_3$ | $4-C_7H_{15}$ | 1.6003 |
| 10 | $CH_3$ | 2-CN | $4-C_7H_{15}$ | 50.0-53.5 |
| 11 | $CH_3$ | $2-NO_2$ | $4-C_7H_{15}$ | 1.5780 |
| 12 | $CH_3$ | $2-CF_3$ | $4-C_7H_{15}$ | 1.5407 |
| 13 | $CH_3$ | 2-Cl | $4-C_8H_{17}$ | 1.5800 |
| 14 | $CH_3$ | $2,6-F_2$ | $4-C_8H_{17}$ | 1.5532 |
| 15 | $CH_3$ | 2-Cl,6-F | $4-C_8H_{17}$ | 1.5652 |
| 16 | $CH_3$ | 2-Cl | $4-C_9H_{19}$ | 1.5766 |
| 17 | $CH_3$ | 2-Cl,6-F | $4-C_9H_{19}$ | 1.5612 |
| 18 | $CH_3$ | $2,6-F_2$ | $4-C_9H_{19}$ | 1.5518 |
| 19 | $CH_3$ | $2,6-Cl_2$ | $4-C_9H_{19}$ | 1.5698 |
| 20 | $CH_3$ | 2-F | $4-C_{10}H_{21}$ | 1.5595 |
| 21 | $CH_3$ | 2-Cl | $4-C_{10}H_{21}$ | 1.5708 |
| 22 | $CH_3$ | 2-Br | $4-C_{10}H_{21}$ | 1.5780 |
| 23 | $CH_3$ | 2-I | $4-C_{10}H_{21}$ | 1.5875 |
| 24 | $CH_3$ | $2-CH_3$ | $4-C_{10}H_{21}$ | 48.0-50.0 |

Table 2

| Compound No. | $R^1$ | Xn | Y | Melting point (°C) or refractive index($n_D^{20}$) |
|---|---|---|---|---|
| 25 | $CH_3$ | 2-$OCH_3$ | 4-$C_{10}H_{21}$ | 1.5649 |
| 26 | $CH_3$ | 2-$SCH_3$ | 4-$C_{10}H_{21}$ | |
| 27 | $CH_3$ | 2-CN | 4-$C_{10}H_{21}$ | 37.0-40.0 |
| 28 | $CH_3$ | 2-$NO_2$ | 4-$C_{10}H_{21}$ | 55.0-58.0 |
| 29 | $CH_3$ | 2-$CF_3$ | 4-$C_{10}H_{21}$ | 56.0-57.0 |
| 30 | $CH_3$ | 2-Cl,6-F | 4-$C_{10}H_{21}$ | 1.5570 |
| 31 | $CH_3$ | 2,6-$F_2$ | 4-$C_{10}H_{21}$ | 1.5482 |
| 32 | $CH_3$ | 2,6-$Cl_2$ | 4-$C_{10}H_{21}$ | 1.5678 |
| 33 | $CH_3$ | 2,4,6-F3 | 4-$C_{10}H_{21}$ | 1.5340 |
| 34 | $CH_3$ | 2-Cl | 4-$C_{11}H_{23}$ | 52.0-54.0 |
| 35 | $CH_3$ | 2-Cl,6-F | 4-$C_{11}H_{23}$ | 1.5495 |
| 36 | $CH_3$ | 2,6-$Cl_2$ | 4-$C_{11}H_{23}$ | 58.0-60.0 |
| 37 | $CH_3$ | 2,6-$F_2$ | 4-$C_{11}H_{23}$ | 1.5437 |
| 38 | $CH_3$ | 2-Cl | 4-$C_{12}H_{25}$ | 62.0-63.0 |
| 39 | $CH_3$ | 2-Cl,6-F | 4-$C_{12}H_{25}$ | 51.0-52.0 |
| 40 | $CH_3$ | 2,6-$F_2$ | 4-$C_{12}H_{25}$ | 43.0-44.5 |
| 41 | $CH_3$ | 2,6-$Cl_2$ | 4-$C_{12}H_{25}$ | 53.0-54.5 |
| 42 | $CH_3$ | 2-Cl | 4-$C_{13}H_{27}$ | 55.0-57.0 |
| 43 | $CH_3$ | 2-Cl,6-F | 4-$C_{13}H_{27}$ | 43.0-47.0 |
| 44 | $CH_3$ | 2,6-$F_2$ | 4-$C_{13}H_{27}$ | 37.0-40.0 |
| 45 | $CH_3$ | 2,6-$Cl_2$ | 4-$C_{13}H_{27}$ | 52.0-55.0 |
| 46 | $CH_3$ | 2-Cl | 4-$C_{14}H_{29}$ | 66.0-67.5 |
| 47 | $CH_3$ | 2-Cl,6-F | 4-$C_{14}H_{29}$ | 56.0-58.0 |
| 48 | $CH_3$ | 2,6-$F_2$ | 4-$C_{14}H_{29}$ | 61.0-62.5 |
| 49 | $CH_3$ | 2,6-$Cl_2$ | 4-$C_{14}H_{29}$ | 47.0-49.0 |
| 50 | $CH_3$ | 2-Cl | 4-$C_{15}H_{31}$ | 62.0-65.0 |
| 51 | $CH_3$ | 2-Cl,6-F | 4-$C_{15}H_{31}$ | 61.0-63.0 |
| 52 | $CH_3$ | 2,6-$F_2$ | 4-$C_{15}H_{31}$ | 54.0-56.0 |

Table 3

| Compound No. | $R^1$ | Xn | Y | Melting point (°C) or refractive index ($n_D^{20}$) |
|---|---|---|---|---|
| 53 | $CH_3$ | 2,6-$Cl_2$ | 4-$C_{15}H_{31}$ | 61.5-64.0 |
| 54 | $CH_3$ | 2-Cl | 4-$C_{16}H_{33}$ | 70.0-73.0 |
| 55 | $CH_3$ | 2-Cl,6-F | 4-$C_{16}H_{33}$ | 65.0-67.0 |
| 56 | $CH_3$ | 2,6-$F_2$ | 4-$C_{16}H_{33}$ | 55.0-57.0 |
| 57 | $CH_3$ | 2,6-$Cl_2$ | 4-$C_{16}H_{33}$ | 69.5-71.0 |
| 58 | $CH_3$ | 2-Cl | 4-$C_{17}H_{35}$ | |
| 59 | $CH_3$ | 2-Cl,6-F | 4-$C_{17}H_{35}$ | |
| 60 | $CH_3$ | 2,6-$F_2$ | 4-$C_{17}H_{35}$ | |
| 61 | $CH_3$ | 2-Cl | 4-$C_{18}H_{37}$ | |
| 62 | $CH_3$ | 2-Cl,6-F | 4-$C_{18}H_{37}$ | |
| 63 | $CH_3$ | 2,6-$F_2$ | 4-$C_{18}H_{37}$ | |
| 64 | $C_2H_5$ | 2-Cl,6-F | 4-$C_{12}H_{25}$ | 43.0-45.0 |
| 65 | $CH(CH_3)_2$ | 2-Cl | 4-$C_{12}H_{25}$ | |
| 66 | $CH(CH_3)_2$ | 2-Cl,6-F | 4-$C_{12}H_{25}$ | 63.0-66.0 |
| 67 | $CH_3$ | 2-Cl | 4-$CH_2CH_2CH_2CH_2CH(CH_3)_2$ | 64.0-67.0 |
| 68 | $CH_3$ | 2-Cl,6-F | 4-$CH_2CH_2CH_2CH_2CH(CH_3)_2$ | 1.5614 |
| 69 | $CH_3$ | 2,6-$F_2$ | 4-$CH_2CH_2CH_2CH_2CH(CH_3)_2$ | 1.5578 |
| 70 | $CH_3$ | 2-Cl | 4-$CH_2CH_2CH_2CH(C_2H_5)CH_3$ | 1.5935 |
| 71 | $CH_3$ | 2-Cl,6-F | 4-$CH_2CH_2CH_2CH(C_2H_5)CH_3$ | 1.5759 |
| 72 | $CH_3$ | 2-Cl | 4-$CH_2CH_2CH(CH_3)CH_2CH_2CH_3$ | 1.5879 |
| 73 | $CH_3$ | 2-Cl,6-F | 4-$CH_2CH_2CH(CH_3)CH_2CH_2CH_3$ | 1.5693 |
| 74 | $CH_3$ | 2-Cl | 4-$CH_2CH_2CH_2C(CH_3)_3$ | |
| 75 | $CH_3$ | 2-Cl,6-F | 4-$CH_2CH_2CH_2C(CH_3)_3$ | |
| 76 | $CH_3$ | 2,6-$F_2$ | 4-$CH_2CH_2CH_2C(CH_3)_3$ | |
| 77 | $CH_3$ | 2-Cl | 4-$OC_8H_{17}$ | 58.0-59.5 |
| 78 | $CH_3$ | 2-Cl | 4-$O(CH_2)_4CH(CH_3)_2$ | |
| 79 | $CH_3$ | 2-Cl,6-F | 4-$O(CH_2)_4CH(CH_3)_2$ | |

Table 4

| Compound No. | $R^1$ | Xn | Y | Melting point (°C) or refractive index ($n_D^{20}$) |
|---|---|---|---|---|
| 80 | $CH_3$ | 2-Cl | 4-$OCH_2$ 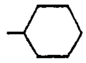 | 83.0-86.0 |
| 81 | $CH_3$ | 2-Cl,6-F | 4-$OCH_2$ 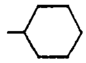 | 83.0-85.0 |
| 82 | $CH_3$ | 2-Cl | 4-$OC_{10}H_{21}$ | 67.5-69.0 |
| 83 | $CH_3$ | 2-Cl,6-F | 4-$OC_{10}H_{21}$ | 55.0-57.0 |
| 84 | $CH_3$ | 2,6-$F_2$ | 4-$OC_{10}H_{21}$ | 1.5399 |
| 85 | $CH_3$ | 2,6-$Cl_2$ | 4-$OC_{10}H_{21}$ | 60.0-64.0 |
| 86 | $CH_3$ | 2-Cl | 4-$OC_{12}H_{25}$ | 73.5-75.0 |
| 87 | $CH_3$ | 2-Cl,6-F | 4-$OC_{12}H_{25}$ | 59.0-61.0 |
| 88 | $CH_3$ | 2-Cl | 4-$SC_8H_{17}$ | 1.6082 |
| 89 | $CH_3$ | 2-Cl,6-F | 4-$SC_8H_{17}$ | 61.0-63.0 |
| 90 | $CH_3$ | 2-Cl | 4-$SOC_8H_{17}$ | 1.5933 |
| 91 | $CH_3$ | 2-Cl | 4-$SO_2C_8H_{17}$ | 1.5855 |
| 92 | $CH_3$ | 2-Cl | 4-$OCH_2CH_2OCH_3$ | 1.6003 |
| 93 | $CH_3$ | 2-Cl | 4-$CH_2OC_4H_9$ | 1.5850 |
| 94 | $CH_3$ | 2-Cl | 4-$CH_2OC_{10}H_{21}$ | |
| 95 | $CH_3$ | 2-Cl,6-F | 4-$CH_2OC_{10}H_{21}$ | |
| 96 | $CH_3$ | 2-Cl,6-F | 4-$CH_2SC_3H_7$ | 1.6023 |
| 97 | $CH_3$ | 2-Cl | 4-$CH{=}CHCH_3$ | 1.6410 |
| 98 | $CH_3$ | 2-Cl | 4-$CH{=}CHC_{10}H_{21}$ | |
| 99 | $CH_3$ | 2-Cl,6-F | 4-$CH{=}CHC_{10}H_{21}$ | |
| 100 | $CH_3$ | 2-Cl | 4-$C{\equiv}CCH_3$ | 93.5-95.0 |
| 101 | $CH_3$ | 2-Cl,6-F | 4-$C{\equiv}CCH_3$ | 124.0-126.5 |
| 102 | $CH_3$ | 2-Cl | 2-$C{\equiv}CC_2H_5$ | 1.6249 |
| 103 | $CH_3$ | 2-Cl | 4-$C{\equiv}CC_2H_5$ | 1.6478 |
| 104 | $CH_3$ | 2,6-$F_2$ | 4-$C{\equiv}CC_2H_5$ | 1.6158 |

Table 5

| Compound No. | $R^1$ | Xn | Y | Melting point (°C) or refractive index ($n_D^{20}$) |
|---|---|---|---|---|
| 105 | $CH_3$ | 2-Cl,6-F | 4-C≡$CC_2H_5$ | 1.6244 |
| 106 | $CH_3$ | 2-Cl | 3-C≡$CC_3H_7$ | 1.6265 |
| 107 | $CH_3$ | 2-Cl | 4-C≡$CC_3H_7$ | 1.5380 |
| 108 | $CH_3$ | 2,6-F2 | 4-C≡$CC_3H_7$ | 1.6018 |
| 109 | $CH_3$ | 2-Cl,6-F | 4-C≡$CC_3H_7$ | 1.6175 |
| 110 | $CH_3$ | 2-Cl | 4-C≡$CCH_2CH(CH_3)_2$ | 82.0-84.0 |
| 111 | $CH_3$ | 2-Cl | 3-C≡$CC_4H_9$ | 1.6191 |
| 112 | $CH_3$ | 2-Cl,6-F | 3-C≡$CC_4H_9$ | 1.6121 |
| 113 | $CH_3$ | 2-Cl | 4-C≡$CC_4H_9$ | 1.6273 |
| 114 | $CH_3$ | 2-Cl,6-F | 4-C≡$CC_4H_9$ | 1.6110 |
| 115 | $CH_3$ | 2,6-$F_2$ | 4-C≡$CC_4H_9$ | |
| 116 | $CH_3$ | 2,6-$Cl_2$ | 4-C≡$CC_4H_9$ | |
| 117 | $CH_3$ | 2-Cl | 3-C≡$CC_5H_{11}$ | 1.6010 |
| 118 | $CH_3$ | 2-Cl,6-F | 3-C≡$CC_5H_{11}$ | 1.5947 |
| 119 | $CH_3$ | 2-Cl | 4-C≡$CC_5H_{11}$ | 1.6224 |
| 120 | $CH_3$ | 2-Cl,6-F | 4-C≡$CC_5H_{11}$ | 1.6052 |
| 121 | $CH_3$ | 2,6-$F_2$ | 4-C≡$CC_5H_{11}$ | |
| 122 | $CH_3$ | 2,6-$Cl_2$ | 4-C≡$CC_5H_{11}$ | |
| 123 | $CH_3$ | 2-Cl,6-F | 4-C≡$CC_6H_{13}$ | |
| 124 | $CH_3$ | 2,6-$F_2$ | 4-C≡$CC_6H_{13}$ | |
| 125 | $CH_3$ | 2,6-$Cl_2$ | 4-C≡$CC_6H_{13}$ | |
| 126 | $CH_3$ | 2-Cl | 4-C≡$CC_8H_{17}$ | 1.5852 |
| 127 | $CH_3$ | 2-Cl,6-F | 4-C≡$CC_8H_{17}$ | 60.5-64.0 |
| 128 | $CH_3$ | 2-Cl | 4 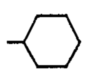 | 79.5-82.0 |
| 129 | $CH_3$ | 2-Cl | 3-$CH_2$ 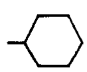 | |

Table 6

| Compound No. | $R^1$ | Xn | Y | Melting point (°C) or refractive index ($n_D^{20}$) |
|---|---|---|---|---|
| 130 | $CH_3$ | 2-Cl | 4-$CH_2CH_2$ 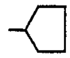 | 116.0-118.0 |
| 131 | $CH_3$ | 2-Cl,6-F | 4-$CH_2CH_2$ 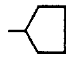 | 88.5-90.0 |
| 132 | $CH_3$ | 2-Cl | 4-$CH_2CH_2$ 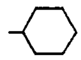 | 90.0-95.0 |
| 133 | $CH_3$ | 2-Cl,6-F | 4-$CH_2CH_2$ 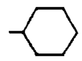 | 105.0-108.0 |
| 134 | $CH_3$ | 2-Cl | 4-$(CH_2)_3$ 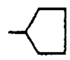 | 65.0-69.0 |
| 135 | $CH_3$ | 2-Cl,6-F | 4-$(CH_2)_3$ 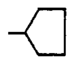 | 53.0-57.0 |
| 136 | $CH_3$ | 2-Cl | 4-$(CH_2)_3$ 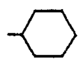 | 118.0-121.0 |
| 137 | $CH_3$ | 2-Cl,6-F | 4-$(CH_2)_3$ 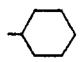 | 100.0-103.0 |
| 138 | $CH_3$ | 2-Cl | 4-CH=CH 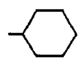 | |
| 139 | $CH_3$ | 2-Cl | 4-C≡C 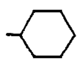 | 104.0-107.0 |
| 140 | $CH_3$ | 2-Cl,6-F | 4-C≡C 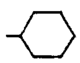 | not measurable |
| 141 | $CH_3$ | 2-Cl | 4-$CH_2CH_2$-$Si(CH_3)_3$ | 79.0-81.0 |
| 142 | $CH_3$ | 2-Cl,6-F | 4-$CH_2CH_2$-$Si(CH_3)_3$ | 1.5728 |
| 143 | $CH_3$ | 2-Cl | 4-O-$CH_2$-$Si(CH_3)_3$ | 55.0-57.0 |
| 144 | $CH_3$ | 2-Cl,6-F | 4-O-$CH_2$-$Si(CH_3)_3$ | 1.5730 |
| 145 | $C_2H_5$ | 2-Cl,6-F | 4-$C_{16}H_{33}$ | 56.0-59.0 |

Table 7

| Compound No. | $R^1$ | Xn | Y | Melting point (°C) or refractive index ($n_D^{20}$) |
|---|---|---|---|---|
| 146 | $CH_3$ | 2,6-$F_2$ | 4-$CH_2CH_2CH_2$- 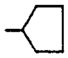 | not measurable |
| 147 | $CH_3$ | 2-Cl | 4-C≡CCH($CH_3$)$CH_2CH_2CH_3$ | 1.6171 |
| 148 | $CH_3$ | 2-Cl | 3-$C_8H_{17}$ | 1.5810 |
| 149 | $CH_3$ | 2-Cl,6-F | 3-$C_8H_{17}$ | 1.5586 |
| 150 | $CH_3$ | 2-Cl | 3-$CH_2CH_2C(CH_3)_3$ | 1.5803 |
| 151 | $CH_3$ | 2-Cl,6-F | 3-$CH_2CH_2C(CH_3)_3$ | 1.5499 |
| 152 | $CH_3$ | 2-Cl | 3-$OC_8H_{17}$ | 1.5789 |
| 153 | $CH_3$ | 2-Cl,6-F | 3-$OC_8H_{17}$ | 1.5559 |
| 154 | $CH_3$ | 2-Cl,6-F | 3-$OC_7H_{15}$ | |
| 155 | $CH_3$ | 2,6-$F_2$ | 3-$OC_7H_{15}$ | |
| 156 | $CH_3$ | 2-Cl,6-F | 3-$OC_9H_{19}$ | |
| 157 | $CH_3$ | 2,6-$F_2$ | 3-$OC_9H_{19}$ | |
| 158 | $CH_3$ | 2-Cl,6-F | 3-$OC_{10}H_{21}$ | |
| 159 | $CH_3$ | 2,6-$F_2$ | 3-$OC_{10}H_{21}$ | |
| 160 | $CH_3$ | 2-Cl,6-F | 3-$OC_{11}H_{23}$ | |
| 161 | $CH_3$ | 2,6-$F_2$ | 3-$OC_{11}H_{23}$ | |
| 162 | $CH_3$ | 2-Cl | 3-$OC_{12}H_{25}$ | 1.5624 |
| 163 | $CH_3$ | 2-Cl,6-F | 3-$OC_{12}H_{25}$ | 1.5491 |
| 164 | $CH_3$ | 2,6-$F_2$ | 3-$OC_{12}H_{25}$ | |

Table 8

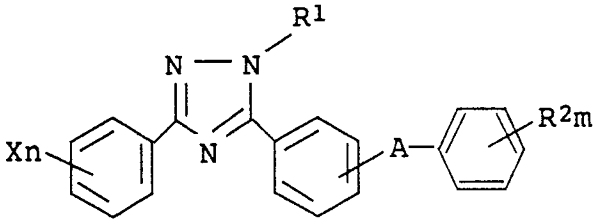

| Compound No. | $R^1$ | Xn | Substitution position | A | $R^2m$ | Melting point (°C) or refractive index ($n_D^{20}$) |
|---|---|---|---|---|---|---|
| 165 | $CH_3$ | 2-Cl | 4- | - | H | 152.0-154.5 |
| 166 | $CH_3$ | 2,6-$F_2$ | 4- | - | 4-$C_3H_7$ | 112.0-116.0 |
| 167 | $CH_3$ | 2-Cl | 4- | - | 4-$C_3H_7$ | 111.5-114.0 |
| 168 | $CH_3$ | 2-Cl,6-F | 4- | - | 4-$C_3H_7$ | 158.0-160.5 |
| 169 | $CH_3$ | 2-Cl | 4- | - | 4-$C_6H_{13}$ | 112.0-114.0 |
| 170 | $CH_3$ | 2-Cl,6-F | 4- | - | 4-$C_6H_{13}$ | 93.0-95.0 |
| 171 | $CH_3$ | 2,6-$F_2$ | 4- | - | 4-$C_6H_{13}$ | 96.0-98.0 |
| 172 | $CH_3$ | 2,6-$Cl_2$ | 4- | - | 4-$C_6H_{13}$ | 96.0-97.5 |
| 173 | $CH_3$ | 2-Cl | 4- | - | 4-Cl | |
| 174 | $CH_3$ | 2-Cl | 4- | - | 4-$OCH_3$ | 137.0-141.0 |
| 175 | $CH_3$ | 2-Cl | 4- | - | 3-$CH_3$ | 137.0-139.0 |
| 176 | $CH_3$ | 2-Cl | 4- | $CH_2$ | H | 68.0-71.0 |
| 177 | $CH_3$ | 2-Cl,6-F | 4- | $CH_2$ | H | 1.6248 |
| 178 | $CH_3$ | 2-Cl | 4- | $CH_2$ | 4-Cl | |
| 179 | $CH_3$ | 2-Cl,6-F | 4- | $CH_2$ | 4-Cl | |
| 180 | $CH_3$ | 2-Cl | 4- | $CH_2$ | 4-$C_4H_9$ | |
| 181 | $CH_3$ | 2-Cl,6-F | 4- | $CH_2$ | 4-$C_4H_9$ | |
| 182 | $CH_3$ | 2-Cl | 4- | $CH_2CH_2$ | H | 68.0-69.0 |
| 183 | $CH_3$ | 2-Cl,6-F | 4- | $CH_2CH_2$ | H | 160.0-162.0 |
| 184 | $CH_3$ | 2-Cl | 4- | $CH_2O$ | H | 99.0-102.0 |
| 185 | $CH_3$ | 2-Cl,6-F | 4- | $CH_2O$ | H | 103.0-106.0 |
| 186 | $CH_3$ | 2-Cl | 4- | $OCH_2$ | H | 83.0-87.0 |
| 187 | $CH_3$ | 2-Cl,6-F | 4- | $OCH_2$ | H | 143.0-153.0 |

Table 9

| Compound No. | $R^1$ | Xn | Substitution position | A | $R^2m$ | Melting point (°C) or refractive index($n_D^{20}$) |
|---|---|---|---|---|---|---|
| 188 | $CH_3$ | 2-Cl | 4- | $CH_2OCH_2$ | H | 87.0-92.0 |
| 189 | $CH_3$ | 2-Cl,6-F | 4- | $CH_2OCH_2$ | H | 93.0-98.0 |
| 190 | $CH_3$ | 2-Cl | 3- | O | H | 1.6354 |
| 191 | $CH_3$ | 2-Cl | 4- | O | H | 106.0-108.0 |
| 192 | $CH_3$ | 2-Cl,6-F | 4- | O | H | 165.0-168.0 |
| 193 | $CH_3$ | 2,6-$F_2$ | 4- | O | H | 85.0-89.0 |
| 194 | $CH_3$ | 2-Cl | 4- | O | 4-$CH_3$ | not measurable |
| 195 | $CH_3$ | 2-Cl,6-F | 4- | O | 4-$CH_3$ | not measurable |
| 196 | $CH_3$ | 2-Cl | 4- | O | 4-$C_4H_9$ | |
| 197 | $CH_3$ | 2-Cl,6-F | 4- | O | 4-$C_4H_9$ | |
| 198 | $CH_3$ | 2-Cl | 4- | O | 2-Cl | 1.6388 |
| 199 | $CH_3$ | 2-Cl,6-F | 4- | O | 2-Cl | 1.6251 |
| 200 | $CH_3$ | 2-Cl | 4- | O | 2-Cl,4-$CF_3$ | |
| 201 | $CH_3$ | 2-Cl,6-F | 4- | O | 2-Cl,4-$CF_3$ | |
| 202 | $CH_3$ | 2-Cl | 4- | - | 4-$CH_3$ | 151.0-154.0 |
| 203 | $CH_3$ | 2-Cl,6-F | 4- | - | 4-$CH_3$ | 207.0-211.0 |
| 204 | $CH_3$ | 2-Cl | 4- | - | 4-$OCF_3$ | 119.0-122.0 |
| 205 | $CH_3$ | 2-Cl,6-F | 4- | - | 4-$OCF_3$ | 114.0-116.0 |
| 206 | $CH_3$ | 2-Cl | 4- | - | 4-$CF_3$ | 155.0-159.0 |
| 207 | $CH_3$ | 2-Cl,6-F | 4- | - | 4-$CF_3$ | 146.0-149.0 |
| 208 | $CH_3$ | 2-Cl | 4- | - | 3,4-$Cl_2$ | |
| 209 | $CH_3$ | 2-Cl,6-F | 4- | - | 3,4-$Cl_2$ | |
| 210 | $CH_3$ | 2-Cl | 4- | - | 2,4-$Cl_2$ | |
| 211 | $CH_3$ | 2-Cl,6-F | 4- | - | 2,4-$Cl_2$ | |
| 212 | $CH_3$ | 2-Cl | 4- | $CH_2O$ | 4-$CH_3$ | 135.0-138.0 |
| 213 | $CH_3$ | 2-Cl,6-F | 4- | $CH_2O$ | 4-$CH_3$ | 149.0-152.0 |
| 214 | $CH_3$ | 2-Cl | 4- | $CH_2O$ | 4-$C_4H_9$ | |
| 215 | $CH_3$ | 2-Cl,6-F | 4- | $CH_2O$ | 4-$C_4H_9$ | |

Table 10

| Compound No. | $R^1$ | Xn | Substitution position | A | $R^2$m refractive | Melting point (°C) or index($n_D^{20}$) |
|---|---|---|---|---|---|---|
| 216 | $CH_3$ | 2-Cl | 4- | $OCH_2$ | 4-$CH_3$ | 108.0-110.0 |
| 217 | $CH_3$ | 2-Cl,6-F | 4- | $OCH_2$ | 4-$CH_3$ | 150.0-155.0 |
| 218 | $CH_3$ | 2-Cl | 4- | $OCH_2$ | 2,3,4,5,6-$F_5$ | |
| 219 | $CH_3$ | 2-Cl,6-F | 4- | $OCH_2$ | 2,3,4,5,6-$F_5$ | |
| 220 | $CH_3$ | 2-Cl | 4- | O | 4-$C_6H_{13}$ | 1.6060 |
| 221 | $CH_3$ | 2-Cl,6-F | 4- | O | 4-$C_6H_{13}$ | 1.5891 |
| 222 | $CH_3$ | 2-Cl | 4- | O | 3,4-$Cl_2$ | 115.0-118.0 |
| 223 | $CH_3$ | 2-Cl,6-F | 4- | O | 3,4-$Cl_2$ | 103.0-106.0 |
| 224 | $CH_3$ | 2-Cl | 4- | O | 2,4-$Cl_2$ | not measurable |
| 225 | $CH_3$ | 2-Cl,6-F | 4- | O | 2,4-$Cl_2$ | not measurable |
| 226 | $CH_3$ | 2-Cl,6-F | 4- | - | 4-$OCH_3$ | 191.0-192.0 |
| 227 | $CH_3$ | 2-Cl | 4- | - | 4-$OC_4H_9$ | 118.0-121.0 |
| 228 | $CH_3$ | 2-Cl,6-F | 4- | - | 4-$OC_4H_9$ | 141.0-144.0 |
| 229 | $CH_3$ | 2-Cl,6-F | 4- | - | 3-$CH_3$ | 131.0-134.0 |
| 230 | $CH_3$ | 2-Cl,6-F | 4- | - | 4-Cl | 105.0-107.0 |
| 231 | $CH_3$ | 2-Cl | 4- | $CH_2CH_2$ | 4-$CH_3$ | 95.0-97.0 |
| 232 | $CH_3$ | 2-Cl,6-F | 4- | $CH_2CH_2$ | 4-$CH_3$ | 188.0-192.0 |
| 233 | $CH_3$ | 2-Cl | 4- | O | 3,5-$Cl_2$ | 105.0-108.0 |
| 234 | $CH_3$ | 2-Cl,6-F | 4- | O | 3,5-$Cl_2$ | 121.0-123.0 |
| 235 | $CH_3$ | 2-Cl,6-F | 4- | O | 4-Cl | not measurable |

Table 11

| Compound No. | $R^1$ | Xn | Substitution position | A | $R^2m$ | Melting point (°C) or refractive index($n_D^{20}$) |
|---|---|---|---|---|---|---|
| 236 | $CH_3$ | 2-Cl | 3- | - | 4-$CF_3$ | |
| 237 | $CH_3$ | 2-Cl,6-F | 3- | - | 4-$CF_3$ | |
| 238 | $CH_3$ | 2,6-$F_2$ | 3- | - | 4-$CF_3$ | |
| 239 | $CH_3$ | 2-Cl,6-F | 3- | O | 4-$CF_3$ | not measurable |
| 240 | $CH_3$ | 2-Cl,6-F | 3- | - | 4-$OCF_3$ | not measurable |
| 241 | $CH_3$ | 2-Cl,6-F | 3- | O | 4-$OCF_3$ | |
| 242 | $CH_3$ | 2-Cl,6-F | 3- | O | 2-Cl,4-$CF_3$ | |
| 243 | $CH_3$ | 2,6-$F_2$ | 4- | - | 4-$CF_3$ | |
| 244 | $CH_3$ | 2-Cl,6-F | 4- | O | 4-$CF_3$ | 114.0-117.0 |
| 245 | $CH_3$ | 2-Cl,6-F | 4- | $CH_2O$ | 4-$CF_3$ | |
| 246 | $CH_3$ | 2-Cl,6-F | 4- | O | 4-$OCF_3$ | 101.0-102.0 |
| 247 | $CH_3$ | 2-Cl,6-F | 4- | O | 3,4-$F_2$ | 95.0-99.0 |
| 248 | $CH_3$ | 2-Cl | 3- | O | 4-$CF_3$ | |
| 249 | $CH_3$ | 2,6-$F_2$ | 3- | O | 4-$CF_3$ | |
| 250 | $CH_3$ | 2-Cl | 3- | S | 4-$CF_3$ | |
| 251 | $CH_3$ | 2,6-$F_2$ | 3- | S | 4-$CF_3$ | |
| 252 | $CH_3$ | 2-Cl,6-F | 3- | S | 4-$CF_3$ | |
| 253 | $CH_3$ | 2,6-$F_2$ | 3- | O | 4-$OCF_3$ | |
| 254 | $CH_3$ | 2,6-$F_2$ | 3- | S | 4-$OCF_3$ | |
| 255 | $CH_3$ | 2-Cl | 4- | O | 4-$CF_3$ | |
| 256 | $CH_3$ | 2,6-$F_2$ | 4- | O | 4-$CF_3$ | |
| 257 | $CH_3$ | 2-Cl,6-F | 4- | O | 4-$CF_3$ | |
| 258 | $CH_3$ | 2,6-$F_2$ | 4- | - | 4-$OCF_3$ | |
| 259 | $CH_3$ | 2-Cl,6-F | 3- | $CH_2$ | 4-$CF_3$ | |
| 260 | $CH_3$ | 2,6-$F_2$ | 3- | $CH_2$ | 4-$CF_3$ | |
| 261 | $CH_3$ | 2-Cl,6-F | 4- | $CH_2$ | 4-$CF_3$ | |
| 262 | $CH_3$ | 2,6-$F_2$ | 4- | $CH_2$ | 4-$CF_3$ | |
| 263 | $CH_3$ | 2-Cl,6-F | 3- | $CH_2CH_2$ | 4-$CF_3$ | |
| 264 | $CH_3$ | 2,6-$F_2$ | 3- | $CH_2CH_2$ | 4-$CF_3$ | |

Table 12

| Compound No. | $R^1$ | Xn | Substitution position | A | $R^2m$ | Melting point (°C) or refractive index($n_D^{20}$) |
|---|---|---|---|---|---|---|
| 265 | $CH_3$ | 2-Cl,6-F | 4- | $CH_2CH_2$ | 4-$CF_3$ | |
| 266 | $CH_3$ | 2,6-$F_2$ | 4- | $CH_2CH_2$ | 4-$CF_3$ | |
| 267 | $CH_3$ | 2-Cl,6-F | 3- | $CH_2$ | 4-$OCF_3$ | |
| 268 | $CH_3$ | 2,6-$F_2$ | 3- | $CH_2$ | 4-$OCF_3$ | |
| 269 | $CH_3$ | 2-Cl,6-F | 4- | $CH_2$ | 4-$OCF_3$ | |
| 270 | $CH_3$ | 2,6-$F_2$ | 4- | $CH_2$ | 4-$OCF_3$ | |
| 271 | $CH_3$ | 2-Cl,6-F | 3- | $CH_2O$ | 4-$CF_3$ | |
| 272 | $CH_3$ | 2,6-$F_2$ | 3- | $CH_2O$ | 4-$CF_3$ | |
| 273 | $CH_3$ | 2-Cl,6-F | 4- | $CH_2O$ | 4-$CF_3$ | |
| 274 | $CH_3$ | 2,6-$F_2$ | 4- | $CH_2O$ | 4-$CF_3$ | |
| 275 | $CH_3$ | 2-Cl,6-F | 3- | $CH_2O$ | 4-$OCF_3$ | |
| 276 | $CH_3$ | 2,6-$F_2$ | 3- | $CH_2O$ | 4-$OCF_3$ | |
| 277 | $CH_3$ | 2-Cl,6-F | 4- | $CH_2O$ | 4-$OCF_3$ | |
| 278 | $CH_3$ | 2,6-$F_2$ | 4- | $CH_2O$ | 4-$OCF_3$ | |
| 279 | $CH_3$ | 2-Cl,6-F | 3- | $OCH_2$ | 4-$CF_3$ | |
| 280 | $CH_3$ | 2,6-$F_2$ | 3- | $OCH_2$ | 4-$CF_3$ | |
| 281 | $CH_3$ | 2-Cl,6-F | 4- | $OCH_2$ | 4-$CF_3$ | |
| 282 | $CH_3$ | 2,6-$F_2$ | 4- | $OCH_2$ | 4-$CF_3$ | |
| 283 | $CH_3$ | 2-Cl,6-F | 3- | O | 2-Cl,4-$CF_3$ | |
| 284 | $CH_3$ | 2,6-$F_2$ | 3- | O | 2-Cl,4-$CF_3$ | |
| 285 | $CH_3$ | 2-Cl,6-F | 3- | $CH_2O$ | 2-Cl,4-$CF_3$ | |
| 286 | $CH_3$ | 2,6-$F_2$ | 3- | $CH_2O$ | 2-Cl,4-$CF_3$ | |
| 287 | $CH_3$ | 2-Cl,6-F | 4- | $CH_2O$ | 2-Cl,4-$CF_3$ | |
| 288 | $CH_3$ | 2,6-$F_2$ | 4- | $CH_2O$ | 2-Cl,4-$CF_3$ | |
| 289 | $CH_3$ | 2-Cl,6-F | 3- | O | 2,6-$Cl_2$,4-$CF_3$ | |
| 290 | $CH_3$ | 2,6-$F_2$ | 3- | O | 2,6-$Cl_2$,4-$CF_3$ | |
| 291 | $CH_3$ | 2-Cl,6-F | 4- | O | 2,6-$Cl_2$,4-$CF_3$ | |
| 292 | $CH_3$ | 2,6-$F_2$ | 4- | O | 2,6-$Cl_2$,4-$CF_3$ | |
| 293 | $CH_3$ | 2-Cl,6-F | 3- | $CH_2O$ | 2,6-$Cl_2$,4-$CF_3$ | |

Table 13

| Compound No. | $R^1$ | Xn | Substitution position | A | $R^2m$ | Melting point (°C) or refractive index($n_D^{20}$) |
|---|---|---|---|---|---|---|
| 294 | $CH_3$ | 2,6-$F_2$ | 3- | $CH_2O$ | 2,6-$Cl_2$,4-$CF_3$ | |
| 295 | $CH_3$ | 2-Cl,6-F | 4- | $CH_2O$ | 2,6-$Cl_2$,4-$CF_3$ | |
| 296 | $CH_3$ | 2,6-$F_2$ | 4- | $CH_2O$ | 2,6-$Cl_2$,4-$CF_3$ | |
| 297 | $CH_3$ | 2-Cl,6-F | 3- | O | 3,5-$(CF_3)_2$ | |
| 298 | $CH_3$ | 2,6-$F_2$ | 3- | O | 3,5-$(CF_3)_2$ | |
| 299 | $CH_3$ | 2-Cl,6-F | 4- | O | 3,5-$(CF_3)_2$ | |
| 300 | $CH_3$ | 2,6-$F_2$ | 4- | O | 3,5-$(CF_3)_2$ | |
| 301 | $CH_3$ | 2-Cl,6-F | 3- | O | 4-Cl,3-$CF_3$ | |
| 302 | $CH_3$ | 2,6-$F_2$ | 3- | O | 4-Cl,3-$CF_3$ | |
| 303 | $CH_3$ | 2-Cl,6-F | 4- | O | 4-Cl,3-$CF_3$ | |
| 304 | $CH_3$ | 2,6-$F_2$ | 4- | O | 4-Cl,3-$CF_3$ | |
| 305 | $CH_3$ | 2-Cl,6-F | 3- | O | 3-F,5-$CF_3$ | |
| 306 | $CH_3$ | 2,6-$F_2$ | 3- | O | 3-F,5-$CF_3$ | |
| 307 | $CH_3$ | 2-Cl,6-F | 4- | O | 3-F,5-$CF_3$ | |
| 308 | $CH_3$ | 2,6-$F_2$ | 4- | O | 3-F,5-$CF_3$ | |
| 309 | $CH_3$ | 2-Cl,6-F | 3- | O | 4-Br | |
| 310 | $CH_3$ | 2,6-$F_2$ | 3- | O | 4-Br | |
| 311 | $CH_3$ | 2-Cl,6-F | 4- | O | 4-Br | |
| 312 | $CH_3$ | 2,6-$F_2$ | 4- | O | 4-Br | |
| 313 | $CH_3$ | 2-Cl,6-F | 4- | $CH_2O$ | 4-Br | |
| 314 | $CH_3$ | 2,6-$F_2$ | 4- | $CH_2O$ | 4-Br | |
| 315 | $CH_3$ | 2-Cl,6-F | 4- | O | 2,4,6-$Br_3$ | |
| 316 | $CH_3$ | 2,6-$F_2$ | 4- | O | 2,4,6-$Br_3$ | |
| 317 | $CH_3$ | 2-Cl,6-F | 4- | $CH_2O$ | 2,4,6-$Br_3$ | |
| 318 | $CH_3$ | 2,6-$F_2$ | 4- | $CH_2O$ | 2,4,6-$Br_3$ | |
| 319 | $CH_3$ | 2-Cl,6-F | 4- | O | 2,4-$Br_2$ | |
| 320 | $CH_3$ | 2,6-$F_2$ | 4- | O | 2,4-$Br_2$ | |
| 321 | $CH_3$ | 2-Cl,6-F | 4- | $CH_2O$ | 2,4-$Br_2$ | |
| 322 | $CH_3$ | 2,6-$F_2$ | 4- | $CH_2O$ | 2,4-$Br_2$ | |

Table 14

| Compound No. | $R^1$ | Xn | Substitution position | A | $R^2m$ | Melting point (°C) or refractive index ($n_D^{20}$) |
|---|---|---|---|---|---|---|
| 323 | $CH_3$ | 2-Cl,6-F | 4- | O | 4-Br,3,5-$(CH_3)_2$ | |
| 324 | $CH_3$ | 2,6-$F_2$ | 4- | O | 4-Br,3,5-$(CH_3)_2$ | |
| 325 | $CH_3$ | 2-Cl,6-F | 4- | $CH_2O$ | 4-Br,3,5-$(CH_3)_2$ | |
| 326 | $CH_3$ | 2,6-$F_2$ | 4- | $CH_2O$ | 4-Br,3,5-$(CH_3)_2$ | |
| 327 | $CH_3$ | 2-Cl,6-F | 4- | O | 4-Br,3-$CH_3$ | |
| 328 | $CH_3$ | 2,6-$F_2$ | 4- | O | 4-Br,3-$CH_3$ | |
| 329 | $CH_3$ | 2-Cl,6-F | 4- | $CH_2O$ | 4-Br,3-$CH_3$ | |
| 330 | $CH_3$ | 2,6-$F_2$ | 4- | $CH_2O$ | 4-Br,3-$CH_3$ | |
| 331 | $CH_3$ | 2-Cl,6-F | 4- | O | 3-Cl,4-F | |
| 332 | $CH_3$ | 2,6-$F_2$ | 4- | O | 3-Cl,4-F | |
| 333 | $CH_3$ | 2-Cl,6-F | 4- | $CH_2O$ | 3-Cl,4-F | |
| 334 | $CH_3$ | 2,6-$F_2$ | 4- | $CH_2O$ | 3-Cl,4-F | |
| 335 | $CH_3$ | 2-Cl,6-F | 4- | O | 2,6-$Cl_2$,5-$CF_3$ | |
| 336 | $CH_3$ | 2,6-$F_2$ | 4- | O | 2,6-$Cl_2$,5-$CF_3$ | |
| 337 | $CH_3$ | 2-Cl,6-F | 4- | $CH_2O$ | 2,6-$Cl_2$,5-$CF_3$ | |
| 338 | $CH_3$ | 2,6-$F_2$ | 4- | $CH_2O$ | 2,6-$Cl_2$,5-$CF_3$ | |
| 339 | $CH_3$ | 2-Cl,6-F | 4- | O | 3,4,5-$F_3$ | |
| 340 | $CH_3$ | 2,6-$F_2$ | 4- | O | 3,4,5-$F_3$ | |
| 341 | $CH_3$ | 2-Cl,6-F | 4- | $CH_2O$ | 3,4,5-$F_3$ | |
| 342 | $CH_3$ | 2,6-$F_2$ | 4- | $CH_2O$ | 3,4,5-$F_3$ | |
| 343 | $CH_3$ | 2-Cl,6-F | 4- | O | 4-F,2-$CF_3$ | |
| 344 | $CH_3$ | 2,6-$F_2$ | 4- | O | 4-F,2-$CF_3$ | |
| 345 | $CH_3$ | 2-Cl,6-F | 4- | $CH_2O$ | 4-F,2-$CF_3$ | |
| 346 | $CH_3$ | 2,6-$F_2$ | 4- | $CH_2O$ | 4-F,2-$CF_3$ | |
| 347 | $CH_3$ | 2-Cl,6-F | 4- | $CH_2O$ | 3,4-$Cl_2$ | |
| 348 | $CH_3$ | 2,6-$F_2$ | 4- | $CH_2O$ | 3,4-$Cl_2$ | |
| 349 | $CH_3$ | 2-Cl,6-F | 4- | $CH_2O$ | 2,4-$Cl_2$ | |
| 350 | $CH_3$ | 2,6-$F_2$ | 4- | $CH_2O$ | 2,4-$Cl_2$ | |
| 351 | $CH_3$ | 2-Cl,6-F | 4- | O | 2,4,5-$Cl_3$ | |

Table 15

| Compound No. | $R^1$ | Xn | Substitution position | A | $R^2m$ | Melting point (°C) or refractive index ($n_D^{20}$) |
|---|---|---|---|---|---|---|
| 352 | $CH_3$ | 2,6-$F_2$ | 4- | O | 2,4,5-$Cl_3$ | |
| 353 | $CH_3$ | 2-Cl,6-F | 4- | $CH_2O$ | 2,4,5-$Cl_3$ | |
| 354 | $CH_3$ | 2,6-$F_2$ | 4- | $CH_2O$ | 2,4,5-$Cl_3$ | |
| 355 | $CH_3$ | 2-Cl,6-F | 4- | O | 2,4,6-$Cl_3$ | |
| 356 | $CH_3$ | 2,6-$F_2$ | 4- | O | 2,4,6-$Cl_3$ | |
| 357 | $CH_3$ | 2-Cl,6-F | 4- | $CH_2O$ | 2,4,6-$Cl_3$ | |
| 358 | $CH_3$ | 2,6-$F_2$ | 4- | $CH_2O$ | 2,4,6-$Cl_3$ | |
| 359 | $CH_3$ | 2-Cl,6-F | 4- | O | 4-Cl,3-$CH_3$ | |
| 360 | $CH_3$ | 2,6-$F_2$ | 4- | O | 4-Cl,3-$CH_3$ | |
| 361 | $CH_3$ | 2-Cl,6-F | 4- | $CH_2O$ | 4-Cl,3-$CH_3$ | |
| 362 | $CH_3$ | 2,6-$F_2$ | 4- | $CH_2O$ | 4-Cl,3-$CH_3$ | |
| 363 | $CH_3$ | 2-Cl,6-F | 4- | O | 4-Cl,3,5-$(CH_3)_2$ | |
| 364 | $CH_3$ | 2,6-$F_2$ | 4- | O | 4-Cl,3,5-$(CH_3)_2$ | |
| 365 | $CH_3$ | 2-Cl,6-F | 4- | $CH_2O$ | 4-Cl,3,5-$(CH_3)_2$ | |
| 366 | $CH_3$ | 2,6-$F_2$ | 4- | $CH_2O$ | 4-Cl,3,5-$(CH_3)_2$ | |
| 367 | $CH_3$ | 2-Cl,6-F | 4- | O | 4-Cl,3-$C_2H_5$ | |
| 368 | $CH_3$ | 2,6-$F_2$ | 4- | O | 4-Cl,3-$C_2H_5$ | |
| 369 | $CH_3$ | 2-Cl,6-F | 4- | $CH_2O$ | 4-Cl,3-$C_2H_5$ | |
| 370 | $CH_3$ | 2,6-$F_2$ | 4- | $CH_2O$ | 4-Cl,3-$C_2H_5$ | |
| 371 | $CH_3$ | 2-Cl,6-F | 4- | O | 4-Cl,3-F | |
| 372 | $CH_3$ | 2,6-$F_2$ | 4- | O | 4-Cl,3-F | |
| 373 | $CH_3$ | 2-Cl,6-F | 4- | $CH_2O$ | 4-Cl,3-F | |
| 374 | $CH_3$ | 2,6-$F_2$ | 4- | $CH_2O$ | 4-Cl,3-F | |
| 375 | $CH_3$ | 2-Cl,6-F | 4- | O | 4-Cl,2-F | |
| 376 | $CH_3$ | 2,6-$F_2$ | 4- | O | 4-Cl,2-F | |
| 377 | $CH_3$ | 2-Cl,6-F | 4- | $CH_2O$ | 4-Cl,2-F | |
| 378 | $CH_3$ | 2,6-$F_2$ | 4- | $CH_2O$ | 4-Cl,2-F | |
| 379 | $CH_3$ | 2-Cl,6-F | 4- | $OCH_2$ | 4-Cl | |
| 380 | $CH_3$ | 2,6-$F_2$ | 4- | $OCH_2$ | 4-Cl | |

Table 16

| Compound No. | $R^1$ | Xn | Substitution position | A | $R^2m$ | Melting point (°C) or refractive index ($n_D^{20}$) |
|---|---|---|---|---|---|---|
| 381 | $CH_3$ | 2-Cl,6-F | 4- | $OCH_2$ | 3,4-$Cl_2$ | |
| 382 | $CH_3$ | 2,6-$F_2$ | 4- | $OCH_2$ | 3,4-$Cl_2$ | |
| 383 | $CH_3$ | 2-Cl,6-F | 4- | $OCH_2$ | 2,4-$Cl_2$ | |
| 384 | $CH_3$ | 2,6-$F_2$ | 4- | $OCH_2$ | 2,4-$Cl_2$ | |
| 385 | $CH_3$ | 2-Cl,6-F | 4- | $OCH_2$ | 4-F | |
| 386 | $CH_3$ | 2,6-$F_2$ | 4- | $OCH_2$ | 4-F | |
| 387 | $CH_3$ | 2-Cl,6-F | 4- | $OCH_2$ | 3,4-$F_2$ | |
| 388 | $CH_3$ | 2,6-$F_2$ | 4- | $OCH_2$ | 3,4-$F_2$ | |
| 389 | $CH_3$ | 2-Cl,6-F | 4- | $OCH_2$ | 2,4-$F_2$ | |
| 390 | $CH_3$ | 2,6-$F_2$ | 4- | $OCH_2$ | 2,4-$F_2$ | |
| 391 | $CH_3$ | 2-Cl,6-F | 4- | $OCH_2$ | 4-Br | |
| 392 | $CH_3$ | 2,6-$F_2$ | 4- | $OCH_2$ | 4-Br | |
| 393 | $CH_3$ | 2-Cl,6-F | 4- | $OCH_2$ | 4-F,3-$CF_3$ | |
| 394 | $CH_3$ | 2,6-$F_2$ | 4- | $OCH_2$ | 4-F,3-$CF_3$ | |
| 395 | $CH_3$ | 2-Cl,6-F | 4- | $OCH_2$ | 4-$OCF_3$ | |
| 396 | $CH_3$ | 2,6-$F_2$ | 4- | $OCH_2$ | 4-$OCF_3$ | |
| 397 | $CH_3$ | 2-Cl,6-F | 4- | $OCH_2$ | 3-$OCF_3$ | |
| 398 | $CH_3$ | 2,6-$F_2$ | 4- | $OCH_2$ | 3-$OCF_3$ | |
| 399 | $CH_3$ | 2-Cl,6-F | 4- | $OCH_2$ | 3,4,5-$F_3$ | |
| 400 | $CH_3$ | 2,6-$F_2$ | 4- | $OCH_2$ | 3,4,5-$F_3$ | |
| 401 | $CH_3$ | 2-Cl,6-F | 4- | $OCH_2$ | 2,4-$(CF_3)_2$ | |
| 402 | $CH_3$ | 2,6-$F_2$ | 4- | $OCH_2$ | 2,4-$(CF_3)_2$ | |
| 403 | $CH_3$ | 2-Cl,6-F | 4- | $OCH_2$ | 2-F,4-$CF_3$ | |
| 404 | $CH_3$ | 2,6-$F_2$ | 4- | $OCH_2$ | 2-F,4-$CF_3$ | |
| 405 | $CH_3$ | 2-Cl,6-F | 4- | $OCH_2$ | 4-F,2-$CF_3$ | |
| 406 | $CH_3$ | 2,6-$F_2$ | 4- | $OCH_2$ | 4-F,2-$CF_3$ | |
| 407 | $CH_3$ | 2,6-$F_2$ | 3- | - | 4-$OCF_3$ | |
| 408 | $CH_3$ | 2-Cl,6-F | 4- | - | 4-Cl,2-$CH_3$ | |
| 409 | $CH_3$ | 2,6-$F_2$ | 4- | - | 4-Cl,2-$CH_3$ | |

Table 17

| Compound No. | $R^1$ | Xn | Substitution position | A | $R^2m$ | Melting point (°C) or refractive index ($n_D^{20}$) |
|---|---|---|---|---|---|---|
| 410 | $CH_3$ | 2-Cl,6-F | 4- | - | 3,5-$Cl_2$ | |
| 411 | $CH_3$ | 2,6-$F_2$ | 4- | - | 3,5-$Cl_2$ | |
| 412 | $CH_3$ | 2-Cl,6-F | 4- | - | 3-Cl,4-F | |
| 413 | $CH_3$ | 2,6-$F_2$ | 4- | - | 3-Cl,4-F | |
| 414 | $CH_3$ | 2-Cl,6-F | 4- | - | 2-Cl,4-$CF_3$ | |
| 415 | $CH_3$ | 2,6-$F_2$ | 4- | - | 2-Cl,4-$CF_3$ | |
| 416 | $CH_3$ | 2-Cl,6-F | 4- | - | 2,4,6-$Cl_3$ | |
| 417 | $CH_3$ | 2,6-$F_2$ | 4- | - | 2,4,6-$Cl_3$ | |
| 418 | $CH_3$ | 2-Cl,6-F | 4- | - | 2,4-$F_2$ | |
| 419 | $CH_3$ | 2,6-$F_2$ | 4- | - | 2,4-$F_2$ | |
| 420 | $CH_3$ | 2-Cl,6-F | 4- | - | 3,4-$F_2$ | |
| 421 | $CH_3$ | 2,6-$F_2$ | 4- | - | 3,4-$F_2$ | |
| 422 | $CH_3$ | 2-Cl,6-F | 4- | - | 2,4-$(CF_3)_2$ | |
| 423 | $CH_3$ | 2,6-$F_2$ | 4- | - | 2,4-$(CF_3)_2$ | |

Table 18

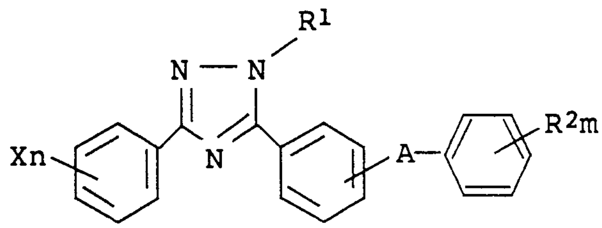

| Compound No. | $R^1$ | Xn | Substitution position | A | $R^2m$ | Melting point (°C) or refractive index ($n_D^{20}$) |
|---|---|---|---|---|---|---|
| 424 | $CH_3$ | 2-Cl | 4- | O | H | |
| 425 | $CH_3$ | 2,6-$F_2$ | 4- | O | H | 122.0-127.0 |
| 426 | $CH_3$ | 2-Cl | 4- | O | 5-$CF_3$ | 107.0-109.0 |
| 427 | $CH_3$ | 2-Cl,6-F | 4- | O | 5-$CF_3$ | 94.0-96.0 |
| 428 | $CH_3$ | 2-Cl | 4- | O | 3-Cl,5-$CF_3$ | not measurable |
| 429 | $CH_3$ | 2-Cl,6-F | 4- | O | 3-Cl,5-$CF_3$ | not measurable |
| 430 | $CH_3$ | 2-Cl | 4- | S | 3-Cl,5-$CF_3$ | 127.0-131.0 |
| 431 | $CH_3$ | 2-Cl | 4- | $CH_2O$ | H | |
| 432 | $CH_3$ | 2-Cl,6-F | 4- | $CH_2O$ | H | |
| 433 | $CH_3$ | 2-Cl,6-F | 2- | O | 5-$CF_3$ | 126.0-129.0 |
| 434 | $CH_3$ | 2-Cl,6-F | 3- | O | H | not measurable |
| 435 | $CH_3$ | 2-Cl,6-F | 3- | O | 5-Cl | not measurable |
| 436 | $CH_3$ | 2,6-$F_2$ | 3- | O | 5-Cl | |
| 437 | $CH_3$ | 2-Cl,6-F | 3- | O | 6-Cl | 124.0-127.0 |
| 438 | $CH_3$ | 2,6-$F_2$ | 3- | O | 6-Cl | |
| 439 | $CH_3$ | 2-Cl,6-F | 3- | O | 4-$CH_3$ | |
| 440 | $CH_3$ | 2-Cl,6-F | 3- | O | 5-$CH_3$ | not measurable |
| 441 | $CH_3$ | 2-Cl,6-F | 3- | O | 6-$CH_3$ | not measurable |
| 442 | $CH_3$ | 2-Cl,6-F | 3- | O | 4-$C_2H_5$ | |
| 443 | $CH_3$ | 2-Cl,6-F | 3- | O | 6-$C_3H_7$ | |
| 444 | $CH_3$ | 2-Cl | 3- | O | 3-$CF_3$ | not measurable |
| 445 | $CH_3$ | 2-Cl,6-F | 3- | O | 3-$CF_3$ | 122.0-124.0 |
| 446 | $CH_3$ | 2,6-$F_2$ | 3- | O | 3-$CF_3$ | |
| 447 | $CH_3$ | 2-Cl,6-F | 3- | O | 4-$CF_3$ | 1.5820 |

Table 19

| Compound No. | $R^1$ | Xn | Substitution position | A | $R^2m$ | Melting point (°C) or refractive index($n_D^{20}$) |
|---|---|---|---|---|---|---|
| 448 | $CH_3$ | 2,6-$F_2$ | 3- | O | 4-$CF_3$ | |
| 449 | $CH_3$ | 2-Cl,6-F | 3- | O | 5-$CF_3$ | |
| 450 | $CH_3$ | 2-Cl | 3- | O | 5-$CF_3$ | not measurable |
| 451 | $CH_3$ | 2-Cl,6-F | 3- | O | 5-$CF_3$ | 65.0-68.0 |
| 452 | $CH_3$ | 2,6-$F_2$ | 3- | O | 5-$CF_3$ | not measurable |
| 453 | $CH_3$ | 2,6-$Cl_2$ | 3- | O | 5-$CF_3$ | |
| 454 | $CH_3$ | 2-Cl,6-F | 3- | S | 5-$CF_3$ | 82.0-86.0 |
| 455 | $CH_3$ | 2-Cl,6-F | 3- | $CH_2$ | 5-$CF_3$ | |
| 456 | $CH_3$ | 2-Cl | 3- | $CH_2O$ | 5-$CF_3$ | |
| 457 | $CH_3$ | 2-Cl,6-F | 3- | $CH_2O$ | 5-$CF_3$ | |
| 458 | $CH_3$ | 2,6-$F_2$ | 3- | $CH_2O$ | 5-$CF_3$ | |
| 459 | $CH_3$ | 2,6-$Cl_2$ | 3- | $CH_2O$ | 5-$CF_3$ | |
| 460 | $CH_3$ | 2-Cl,6-F | 3- | $C_2H_4O$ | 5-$CF_3$ | |
| 461 | $CH_3$ | 2-Cl,6-F | 3- | O | 6-$CF_3$ | 98.0-102.0 |
| 462 | $CH_3$ | 2,6-$F_2$ | 3- | O | 6-$CF_3$ | |
| 463 | $CH_3$ | 2-Cl,6-F | 3- | O | 5-Cl,3-$CF_3$ | |
| 464 | $CH_3$ | 2,6-$F_2$ | 3- | O | 5-Cl,3-$CF_3$ | |
| 465 | $CH_3$ | 2-Cl | 3- | O | 5-Cl,3-$CF_3$ | 71.0-73.0 |
| 466 | $CH_3$ | 2-Cl,6-F | 3- | O | 5-Cl,3-$CF_3$ | 109.0-111.0 |
| 467 | $CH_3$ | 2,6-$F_2$ | 3- | O | 5-Cl,3-$CF_3$ | |
| 468 | $CH_3$ | 2-Cl | 3- | O | 3-Cl,5-$CF_3$ | not measurable |
| 469 | $CH_3$ | 2-Cl,6-F | 3- | O | 3-Cl,5-$CF_3$ | not measurable |
| 470 | $CH_3$ | 2,6-$F_2$ | 3- | O | 3-Cl,5-$CF_3$ | |
| 471 | $CH_3$ | 2-Cl,6-F | 3- | O | 3,5-$(CF_3)_2$ | 91.0-95.0 |
| 472 | $CH_3$ | 2,6-$F_2$ | 3- | O | 3,5-$(CF_3)_2$ | |
| 473 | $CH_3$ | 2-Cl,6-F | 3- | O | 6-Cl,5-$CF_3$ | not measurable |
| 474 | $CH_3$ | 2,6-$F_2$ | 3- | O | 6-Cl,5-$CF_3$ | |
| 475 | $CH_3$ | 2-Cl,6-F | 3- | O | 4,5-$(CF_3)_2$ | 122.0-126.0 |
| 476 | $CH_3$ | 2,6-$F_2$ | 3- | O | 4,5-$(CF_3)_2$ | |

Table 20

| Compound No. | $R^1$ | Xn | Substitution position | A | $R^2m$ | Melting point (°C) or refractive index($n_D^{20}$) |
|---|---|---|---|---|---|---|
| 477 | $CH_3$ | 2-Cl,6-F | 3- | O | 6-Cl,4-$CF_3$ | not measurable |
| 478 | $CH_3$ | 2,6-$F_2$ | 3- | O | 6-Cl,4-$CF_3$ | |
| 479 | $CH_3$ | 2-Cl,6-F | 3- | O | 4,6-$(CF_3)_2$ | 1.5453 |
| 480 | $CH_3$ | 2,6-$F_2$ | 3- | O | 4,6-$(CF_3)_2$ | |
| 481 | $CH_3$ | 2-Cl,6-F | 3- | O | 6-$CH_3$,4-$CF_3$ | 121.0-123.0 |
| 482 | $CH_3$ | 2,6-$F_2$ | 3- | O | 6-$CH_3$,4-$CF_3$ | |
| 483 | $CH_3$ | 2-Cl,6-F | 4- | O | 5-Cl | 136.0-139.0 |
| 484 | $CH_3$ | 2,6-$F_2$ | 4- | O | 5-Cl | |
| 485 | $CH_3$ | 2-Cl,6-F | 4- | O | 6-Cl | 134.0-136.0 |
| 486 | $CH_3$ | 2,6-$F_2$ | 4- | O | 6-Cl | |
| 487 | $CH_3$ | 2-Cl,6-F | 4- | O | 4-$CH_3$ | 136.0-140.0 |
| 488 | $CH_3$ | 2-Cl,6-F | 4- | O | 4-$C_2H_5$ | |
| 489 | $CH_3$ | 2-Cl,6-F | 4- | O | 5-$CH_3$ | 154.0-157.0 |
| 490 | $CH_3$ | 2-Cl,6-F | 4- | O | 6-$CH_3$ | not measurable |
| 491 | $CH_3$ | 2-Cl,6-F | 4- | O | 6-$C_3H_7$ | |
| 492 | $CH_3$ | 2-Cl,6-F | 4- | O | 3-$CF_3$ | 158.0-159.9 |
| 493 | $CH_3$ | 2,6-$F_2$ | 4- | O | 3-$CF_3$ | |
| 494 | $CH_3$ | 2-Cl,6-F | 4- | O | 4-$CF_3$ | 110.0-114.0 |
| 495 | $CH_3$ | 2,6-$F_2$ | 4- | O | 4-$CF_3$ | |
| 496 | $CH_3$ | 2-Cl,6-F | 4- | - | 5-$CF_3$ | |
| 497 | $CH_3$ | 2,6-$F_2$ | 4- | - | 5-$CF_3$ | |
| 498 | $C_2H_5$ | 2-Cl,6-F | 4- | O | 5-$CF_3$ | not measurable |
| 499 | $CH(CH_3)_2$ | 2-Cl,6-F | 4- | O | 5-$CF_3$ | not measurable |
| 500 | $CH_3$ | 2,6-$F_2$ | 4- | O | 5-$CF_3$ | 127.0-131.0 |
| 501 | $CH_3$ | 2,6-$Cl_2$ | 4- | O | 5-$CF_3$ | 127.0-130.0 |
| 502 | $C_6H_{13}$ | 2-Cl,6-F | 4- | O | 5-$CF_3$ | 1.5573 |
| 503 | $CH_3$ | 2-Cl | 4- | S | 5-$CF_3$ | not measurable |
| 504 | $CH_3$ | 2-Cl,6-F | 4- | S | 5-$CF_3$ | 111.0-115.0 |
| 505 | $CH_3$ | 2,6-$F_2$ | 4- | S | 5-$CF_3$ | |

Table 21

| Compound No. | $R^1$ | Xn | Substitution position | A | $R^2m$ | Melting point (°C) or refractive index($n_D^{20}$) |
|---|---|---|---|---|---|---|
| 506 | | | | | | |
| 507 | $CH_3$ | 2-Cl,6-F | 4- | $CH_2$ | 5-$CF_3$ | |
| 508 | $CH_3$ | 2,6-$F_2$ | 4- | $CH_2$ | 5-$CF_3$ | |
| 509 | $CH_3$ | 2-Cl | 4- | $CH_2O$ | 5-$CF_3$ | |
| 510 | $CH_3$ | 2-Cl,6-F | 4- | $CH_2O$ | 5-$CF_3$ | 1.5859 |
| 511 | $CH_3$ | 2,6-$F_2$ | 4- | $CH_2O$ | 5-$CF_3$ | |
| 512 | $CH_3$ | 2,6-$Cl_2$ | 4- | $CH_2O$ | 5-$CF_3$ | |
| 513 | $CH_3$ | 2-Cl,6-F | 4- | $C_2H_4O$ | 5-$CF_3$ | |
| 514 | $CH_3$ | 2-Cl,6-F | 4- | O | 6-$CF_3$ | 97.0-101.0 |
| 515 | $CH_3$ | 2-Cl,6-F | 4- | O | 3,5-$Cl_2$ | |
| 516 | $CH_3$ | 2-Cl,6-F | 4- | O | 5-Cl,3-$CF_3$ | not measurable |
| 517 | $CH_3$ | 2,6-$F_2$ | 4- | O | 5-Cl,3-$CF_3$ | |
| 518 | $CH_3$ | 2-Cl,6-F | 4- | S | 3-Cl,5-$CF_3$ | not measurable |
| 519 | $CH_3$ | 2,6-$F_2$ | 4- | S | 3-Cl,5-$CF_3$ | |
| 520 | $CH_3$ | 2-Cl,6-F | 4- | $CH_2O$ | 3-Cl,5-$CF_3$ | 1.5778 |
| 521 | $CH_3$ | 2,6-$F_2$ | 4- | $CH_2O$ | 3-Cl,5-$CF_3$ | |
| 522 | $CH_3$ | 2-Cl,6-F | 4- | O | 3,5-$(CF_3)_2$ | 85.0-89.0 |
| 523 | $CH_3$ | 2,6-$F_2$ | 4- | O | 3,5-$(CF_3)_2$ | |
| 524 | $CH_3$ | 2-Cl,6-F | 4- | O | 6-Cl,5-$CF_3$ | 108.0-112.0 |
| 525 | $CH_3$ | 2,6-$F_2$ | 4- | O | 6-Cl,5-$CF_3$ | |
| 526 | $CH_3$ | 2-Cl,6-F | 4- | O | 4,5-$(CF_3)_2$ | 158.0-160.0 |
| 527 | $CH_3$ | 2,6-$F_2$ | 4- | O | 4,5-$(CF_3)_2$ | |
| 528 | $CH_3$ | 2-Cl,6-F | 4- | O | 6-Cl,4-$CF_3$ | not measurable |
| 529 | $CH_3$ | 2,6-$F_2$ | 4- | O | 6-Cl,4-$CF_3$ | |
| 530 | $CH_3$ | 2-Cl,6-F | 4- | O | 4,6-$(CF_3)_2$ | 125.0-129.0 |
| 531 | $CH_3$ | 2,6-$F_2$ | 4- | O | 4,6-$(CF_3)_2$ | |
| 532 | $CH_3$ | 2-Cl,6-F | 4- | O | 6-$CH_3$,4-$CF_3$ | 98.0-101.0 |
| 533 | $CH_3$ | 2,6-$F_2$ | 4- | O | 6-$CH_3$,4-$CF_3$ | |
| Compound No. 506 deleted | | | | | | |

Table 22

| Compound No. | $R^1$ | Xn | Substitution position | A | $R^2m$ | Melting point (°C) or refractive index($n_D^{20}$) |
|---|---|---|---|---|---|---|
| 534 | $CH_3$ | 2,6-$F_2$ | 3- | - | 5-$CF_3$ | |
| 535 | $CH_3$ | 2,6-$F_2$ | 3- | S | 5-$CF_3$ | |
| 536 | $CH_3$ | 2,6-$F_2$ | 3- | $CH_2$ | 5-$CF_3$ | |
| 537 | $CH_3$ | 2,6-$Cl_2$ | 3- | O | 3-Cl,5-$CF_3$ | |
| 538 | $CH_3$ | 2-Cl | 3- | S | 3-Cl,5-$CF_3$ | |
| 539 | $CH_3$ | 2-Cl,6-F | 3- | S | 3-Cl,5-$CF_3$ | |
| 540 | $CH_3$ | 2,6-$F_2$ | 3- | S | 3-Cl,5-$CF_3$ | |
| 541 | $CH_3$ | 2,6-$Cl_2$ | 3- | S | 3-Cl,5-$CF_3$ | |
| 542 | $CH_3$ | 2-Cl | 3- | $CH_2O$ | 3-Cl,5-$CF_3$ | |
| 543 | $CH_3$ | 2-Cl,6-F | 3- | $CH_2O$ | 3-Cl,5-$CF_3$ | |
| 544 | $CH_3$ | 2,6-$F_2$ | 3- | $CH_2O$ | 3-Cl,5-$CF_3$ | |
| 545 | $CH_3$ | 2,6-$Cl_2$ | 3- | $CH_2O$ | 3-Cl,5-$CF_3$ | |
| 546 | $CH_3$ | 2-Cl,6-F | 3- | $CH_2O$ | 3,5-$(CF_3)_2$ | |
| 547 | $CH_3$ | 2,6-$F_2$ | 3- | $CH_2O$ | 3,5-$(CF_3)_2$ | |
| 548 | $CH_3$ | 2-Cl,6-F | 3- | $CH_2O$ | 4,5-$(CF_3)_2$ | |
| 549 | $CH_3$ | 2,6-$F_2$ | 3- | $CH_2O$ | 4,5-$(CF_3)_2$ | |
| 550 | $CH_3$ | 2-Cl,6-F | 3- | $CH_2O$ | 4,6-$(CF_3)_2$ | |
| 551 | $CH_3$ | 2,6-$F_2$ | 3- | $CH_2O$ | 4,6-$(CF_3)_2$ | |
| 552 | $CH_3$ | 2-Cl,6-F | 3- | $CH_2O$ | 6-$CH_3$,4-$CF_3$ | |
| 553 | $CH_3$ | 2,6-$F_2$ | 3- | $CH_2O$ | 6-$CH_3$,4-$CF_3$ | |
| 554 | $CH_3$ | 2-Cl,6-F | 3- | $CH_2O$ | 5-Cl | |
| 555 | $CH_3$ | 2,6-$F_2$ | 3- | $CH_2O$ | 5-Cl | |
| 556 | $CH_3$ | 2-Cl,6-F | 3- | $CH_2O$ | 5-$CH_3$ | |
| 557 | $CH_3$ | 2,6-$F_2$ | 3- | $CH_2O$ | 5-$CH_3$ | |
| 558 | $CH_3$ | 2-Cl,6-F | 3- | $CH_2O$ | 3,5-$Cl_2$ | |
| 559 | $CH_3$ | 2,6-$F_2$ | 3- | $CH_2O$ | 3,5-$Cl_2$ | |
| 560 | $CH_3$ | 2-Cl,6-F | 3- | $CH_2O$ | 5-Cl,3-$CF_3$ | |
| 561 | $CH_3$ | 2,6-$F_2$ | 3- | $CH_2O$ | 5-Cl,3-$CF_3$ | |
| 562 | $CH_3$ | 2-Cl,6-F | 3- | $CH_2O$ | 6-Cl,5-$CF_3$ | |

Table 23

| Compound No. | $R^1$ | Xn | Substitution position | A | $R^2m$ | Melting point (°C) or refractive index($n_D^{20}$) |
|---|---|---|---|---|---|---|
| 563 | $CH_3$ | 2,6-$F_2$ | 3- | $CH_2O$ | 6-Cl,5-$CF_3$ | |
| 564 | $CH_3$ | 2-Cl,6-F | 3- | $CH_2O$ | 6-Cl,4-$CF_3$ | |
| 565 | $CH_3$ | 2,6-$F_2$ | 3- | $CH_2O$ | 6-Cl,4-$CF_3$ | |
| 566 | $CH_3$ | 2-Cl,6-F | 3- | O | 3,5-$Cl_2$ | |
| 567 | $CH_3$ | 2,6-$F_2$ | 3- | O | 3,5-$Cl_2$ | |
| 568 | $CH_3$ | 2-Cl,6-F | 4- | $CH_2O$ | 5-Cl | |
| 569 | $CH_3$ | 2,6-$F_2$ | 4- | $CH_2O$ | 5-Cl | |
| 570 | $CH_3$ | 2-Cl,6-F | 4- | $CH_2O$ | 6-Cl | |
| 571 | $CH_3$ | 2,6-$F_2$ | 4- | $CH_2O$ | 6-Cl | |
| 572 | $CH_3$ | 2-Cl,6-F | 4- | $CH_2O$ | 4-$CH_3$ | |
| 573 | $CH_3$ | 2,6-$F_2$ | 4- | $CH_2O$ | 4-$CH_3$ | |
| 574 | $CH_3$ | 2-Cl,6-F | 4- | $CH_2O$ | 5-$CH_3$ | |
| 575 | $CH_3$ | 2,6-$F_2$ | 4- | $CH_2O$ | 5-$CH_3$ | |
| 576 | $CH_3$ | 2-Cl,6-F | 4- | $CH_2O$ | 6-$CH_3$ | |
| 577 | $CH_3$ | 2,6-$F_2$ | 4- | $CH_2O$ | 6-$CH_3$ | |
| 578 | $CH_3$ | 2-Cl,6-F | 4- | $CH_2O$ | 3-$CF_3$ | |
| 579 | $CH_3$ | 2,6-$F_2$ | 4- | $CH_2O$ | 3-$CF_3$ | |
| 580 | $CH_3$ | 2-Cl,6-F | 4- | $CH_2O$ | 4-$CF_3$ | |
| 581 | $CH_3$ | 2,6-$F_2$ | 4- | $CH_2O$ | 4-$CF_3$ | |
| 582 | $CH_3$ | 2-Cl,6-F | 4- | $CH_2O$ | 6-$CF_3$ | |
| 583 | $CH_3$ | 2,6-$F_2$ | 4- | $CH_2O$ | 6-$CF_3$ | |
| 584 | $CH_3$ | 2-Cl | 4- | $CH_2O$ | 3,5-$Cl_2$ | |
| 585 | $CH_3$ | 2-Cl,6-F | 4- | $CH_2O$ | 3,5-$Cl_2$ | |
| 586 | $CH_3$ | 2,6-$F_2$ | 4- | $CH_2O$ | 3,5-$Cl_2$ | |
| 587 | $CH_3$ | 2,6-$Cl_2$ | 4- | $CH_2O$ | 3,5-$Cl_2$ | |
| 588 | $CH_3$ | 2-Cl | 4- | $CH_2O$ | 5-Cl,3-$CF_3$ | |
| 589 | $CH_3$ | 2-Cl,6-F | 4- | $CH_2O$ | 5-Cl,3-$CF_3$ | |
| 590 | $CH_3$ | 2,6-$F_2$ | 4- | $CH_2O$ | 5-Cl,3-$CF_3$ | |
| 591 | $CH_3$ | 2,6-$Cl_2$ | 4- | $CH_2O$ | 5-Cl,3-$CF_3$ | |

Table 24

| Compound No. | $R^1$ | Xn | Substitution position | A | $R^2m$ | Melting point (°C) or refractive index ($n_D^{20}$) |
|---|---|---|---|---|---|---|
| 592 | $CH_3$ | 2-Cl | 4- | $CH_2O$ | 3-Cl,5-$CF_3$ | |
| 593 | $CH_3$ | 2,6-$Cl_2$ | 4- | $CH_2O$ | 3-Cl,5-$CF_3$ | |
| 594 | $CH_3$ | 2-Cl | 4- | $CH_2O$ | 3,5-$(CF_3)_2$ | |
| 595 | $CH_3$ | 2-Cl,6-F | 4- | $CH_2O$ | 3,5-$(CF_3)_2$ | |
| 596 | $CH_3$ | 2,6-$F_2$ | 4- | $CH_2O$ | 3,5-$(CF_3)_2$ | |
| 597 | $CH_3$ | 2,6-$Cl_2$ | 4- | $CH_2O$ | 3,5-$(CF_3)_2$ | |
| 598 | $CH_3$ | 2-Cl | 4- | $CH_2O$ | 6-Cl,5-$CF_3$ | |
| 599 | $CH_3$ | 2-Cl,6-F | 4- | $CH_2O$ | 6-Cl,5-$CF_3$ | |
| 600 | $CH_3$ | 2,6-$F_2$ | 4- | $CH_2O$ | 6-Cl,5-$CF_3$ | |
| 601 | $CH_3$ | 2,6-$Cl_2$ | 4- | $CH_2O$ | 6-Cl,5-$CF_3$ | |
| 602 | $CH_3$ | 2-Cl | 4- | $CH_2O$ | 4,5-$(CF_3)_2$ | |
| 603 | $CH_3$ | 2-Cl,6-F | 4- | $CH_2O$ | 4,5-$(CF_3)_2$ | |
| 604 | $CH_3$ | 2,6-$F_2$ | 4- | $CH_2O$ | 4,5-$(CF_3)_2$ | |
| 605 | $CH_3$ | 2,6-$Cl_2$ | 4- | $CH_2O$ | 4,5-$(CF_3)_2$ | |
| 606 | $CH_3$ | 2-Cl | 4- | $CH_2O$ | 6-Cl,4-$CF_3$ | |
| 607 | $CH_3$ | 2-Cl,6-F | 4- | $CH_2O$ | 6-Cl,4-$CF_3$ | |
| 608 | $CH_3$ | 2,6-$F_2$ | 4- | $CH_2O$ | 6-Cl,4-$CF_3$ | |
| 609 | $CH_3$ | 2,6-$Cl_2$ | 4- | $CH_2O$ | 6-Cl,4-$CF_3$ | |
| 610 | $CH_3$ | 2-Cl | 4- | $CH_2O$ | 4,6-$(CF_3)_2$ | |
| 611 | $CH_3$ | 2-Cl,6-F | 4- | $CH_2O$ | 4,6-$(CF_3)_2$ | |
| 612 | $CH_3$ | 2,6-$F_2$ | 4- | $CH_2O$ | 4,6-$(CF_3)_2$ | |
| 613 | $CH_3$ | 2,6-$Cl_2$ | 4- | $CH_2O$ | 4,6-$(CF_3)_2$ | |
| 614 | $CH_3$ | 2-Cl | 4- | $CH_2O$ | 6-$CH_3$,4-$CF_3$ | |
| 615 | $CH_3$ | 2-Cl,6-F | 4- | $CH_2O$ | 6-$CH_3$,4-$CF_3$ | |
| 616 | $CH_3$ | 2,6-$F_2$ | 4- | $CH_2O$ | 6-$CH_3$,4-$CF_3$ | |
| 617 | $CH_3$ | 2,6-$Cl_2$ | 4- | $CH_2O$ | 6-$CH_3$,4-$CF_3$ | |
| 618 | $CH_3$ | 2-Cl,6-F | 4- | $CH_2CH_2O$ | 3-Cl,5-$CF_3$ | |
| 619 | $CH_3$ | 2,6-$F_2$ | 4- | $CH_2CH_2O$ | 3-Cl,5-$CF_3$ | |
| 620 | $CH_3$ | 2-Cl,6-F | 4- | $CH_2CH_2O$ | 3,5-$(CF_3)_2$ | |

Table 25

| Compound No. | $R^1$ | Xn | Substitution position | A | $R^2m$ | Melting point (°C) or refractive index ($n_D^{20}$) |
|---|---|---|---|---|---|---|
| 621 | $CH_3$ | 2,6-$F_2$ | 4- | $CH_2CH_2O$ | 3,5-$(CF_3)_2$ | |
| 622 | $CH_3$ | 2-Cl,6-F | 4- | $CH_2CH_2O$ | 6-Cl,5-$CF_3$ | |
| 623 | $CH_3$ | 2,6-$F_2$ | 4- | $CH_2CH_2O$ | 6-Cl,5-$CF_3$ | |
| 624 | $CH_3$ | 2-Cl,6-F | 4- | $CH_2CH_2O$ | 5-Cl,3-$CF_3$ | |
| 625 | $CH_3$ | 2,6-$F_2$ | 4- | $CH_2CH_2O$ | 5-Cl,3-$CF_3$ | |
| 626 | $CH_3$ | 2-Cl,6-F | 4- | $CH_2CH_2O$ | 4,5-$(CF_3)_2$ | |
| 627 | $CH_3$ | 2,6-$F_2$ | 4- | $CH_2CH_2O$ | 4,5-$(CF_3)_2$ | |
| 628 | $CH_3$ | 2-Cl,6-F | 4- | $CH_2CH_2O$ | 6-Cl,4-$CF_3$ | |
| 629 | $CH_3$ | 2,6-$F_2$ | 4- | $CH_2CH_2O$ | 6-Cl,4-$CF_3$ | |
| 630 | $CH_3$ | 2-Cl,6-F | 4- | $CH_2CH_2O$ | 4,6-$(CF_3)_2$ | |
| 631 | $CH_3$ | 2,6-$F_2$ | 4- | $CH_2CH_2O$ | 4,6-$(CF_3)_2$ | |
| 632 | $CH_3$ | 2-Cl,6-F | 4- | $CH_2CH_2O$ | 6-$CH_3$,4-$CF_3$ | |
| 633 | $CH_3$ | 2,6-$F_2$ | 4- | $CH_2CH_2O$ | 6-$CH_3$,4-$CF_3$ | |
| 634 | $CH_3$ | 2-Cl,6-F | 4- | $CH_2CH_2O$ | 3,5-$Cl_2$ | |
| 635 | $CH_3$ | 2,6-$F_2$ | 4- | $CH_2CH_2O$ | 3,5-$Cl_2$ | |
| 636 | $CH_3$ | 2-Cl,6-F | 4- | S | 3,5-$Cl_2$ | |
| 637 | $CH_3$ | 2,6-$F_2$ | 4- | S | 3,5-$Cl_2$ | |
| 638 | $CH_3$ | 2-Cl,6-F | 4- | S | 5-Cl,3-$CF_3$ | |
| 639 | $CH_3$ | 2,6-$F_2$ | 4- | S | 5-Cl,3-$CF_3$ | |
| 640 | $CH_3$ | 2-Cl,6-F | 4- | S | 3,5-$(CF_3)_2$ | |
| 641 | $CH_3$ | 2,6-$F_2$ | 4- | S | 3,5-$(CF_3)_2$ | |
| 642 | $CH_3$ | 2-Cl,6-F | 4- | S | 6-Cl,5-$CF_3$ | |
| 643 | $CH_3$ | 2,6-$F_2$ | 4- | S | 6-Cl,5-$CF_3$ | |
| 644 | $CH_3$ | 2-Cl,6-F | 4- | S | 4,5-$(CF_3)_2$ | |
| 645 | $CH_3$ | 2,6-$F_2$ | 4- | S | 4,5-$(CF_3)_2$ | |
| 646 | $CH_3$ | 2-Cl,6-F | 4- | S | 4,6-$(CF_3)_2$ | |
| 647 | $CH_3$ | 2,6-$F_2$ | 4- | S | 4,6-$(CF_3)_2$ | |

The compounds according to the invention can be produced by the following methods. However, it is not intended to restrict the invention to these methods.

Production Method A

The compound of the general formula [I] according to the invention can be obtained by reacting an alkyl N-acyl(thio) imidate derivative of a general formula [II] with a hydrazine derivative of a general formula [III] in an inert solvent according to the following reaction formula (1):

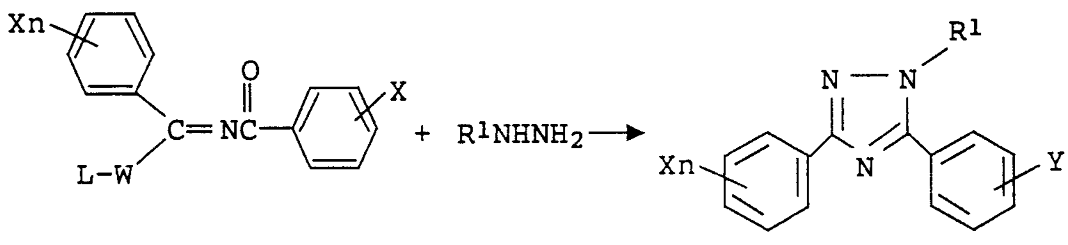

[ II ] [ III ] [ I ]

(wherein W is a sulfur atom or an oxygen atom, L is an alkyl group having a carbon number of 1-4 and $R^1$, X, n and Y have the same meaning as mentioned above).

As the solvent, use may be made of any solvent not obstruction the reaction, which includes, for example, an alcohol such as methanol, ethanol or the like; an ether such as diethyl ether, tetrahydrofuran, dioxane, diglyme or the like; an aromatic hydrocarbon such as benzene, toluene, chlorobenzene or the like; an aliphatic hydrocarbon such as pentane, hexane, petroleum ether or the like; a halogenated hydrocarbon such as dichloromethane, dichloroethane, chloroform, carbon tetrachloride or the like; a nitrile such as acetonitrile or the like; an aprotic polar solvent such as N,N-dimethylformamide, N,N-dimethylacetamide, dimethylsulfoxide or the like; water and a mixture thereof.

In general, the compound of the general formula [III] is used in an amount of 1.0-5.0 moles per 1 mole of the compound of the general formula [II].

The reaction temperature is optional within a range of 0°C to a boiling point of the solvent, but is preferably 0°C-50°C. The reaction time is dependent upon the kind of compounds used, but is usually 1-72 hours.

A concrete example of this reaction is disclosed, for example, in Synthesis, page 483 (1983).

The compound of the general formula [II] as a starting material can be produced by the following method.

Production Method B

The compound of the general formula [II] can be obtained by reacting compounds of general formulae [IV] and [V] in an inert solvent in the presence of a base according to the following reaction formula (2):

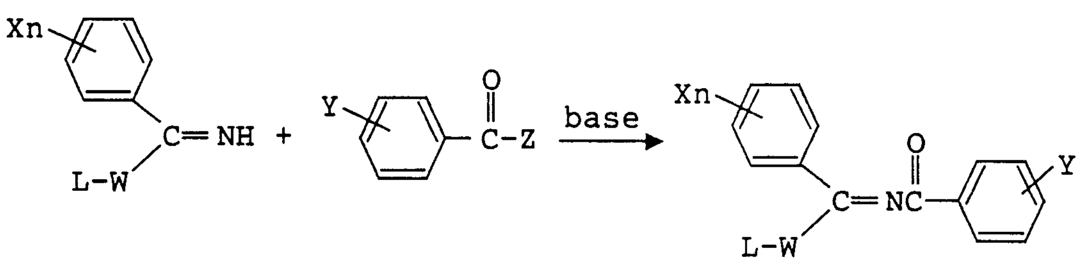


[ IV ] [ V ] [ II ]

(wherein a derivative of the general formula [IV] may be an acid addition salt (e.g. a salt with boron tetrafluoride, hydrogen chloride, hydrogen bromide, hydrogen iodide or the like), Z is a halogen atom, and L, W, X, n and Y have the same meaning as mentioned above).

As the base, use may be made of an inorganic base such as sodium carbonate, potassium carbonate, sodium hydrogen carbonate, sodium hydroxide, potassium hydroxide or the like; and an organic base such as diethylamine, triethylamine, diisopropylethylamine, pyridine, 4-N,N-dimethylamino pyridine or the like.

As the solvent, use may be made of a ketone such as acetone, methyl ethyl ketone or the like; an ether such as diethyl ether, tetrahydrofuran, dioxane, diglyme or the like; an aromatic hydrocarbon such as benzene, toluene, chlorobenzene or the like; an aliphatic hydrocarbon such as pentane, hexane, petroleum ether or the like; a halogenated hydrocarbon such as dichloromethane, dichloroethane, chloroform, carbon tetrachloride or the like; a nitrile such as acetonitrile or the like; an aprotic polar solvent such as N,N-dimethylformamide, N,N-dimethylacetamide, dimethylsulfoxide or the like; and a mixture thereof.

In general, the compound of the general formula [V] is used in an amount of 0.8-1.3 moles per 1 mole of the compound of the general formula [IV]. The amount of the base used is 1.0-2.0 moles per 1 mole of the compound of the general formula [IV].

The reaction time is dependent upon the kind of the compounds used, but is usually within a range of 1-24 hours. The reaction temperature is within a range of 0°C to a boiling point of the solvent.

Production Method C

The compound of the general formula [I] according to the invention can be obtained by reacting an N-(phenylsulfonyl) benzohydrazonoyl chloride derivative of a general formula [VI] with a benzonitrile derivative of a general formula [VII] in an inert solvent in the presence of Lewis acid according to the following reaction formula (3):

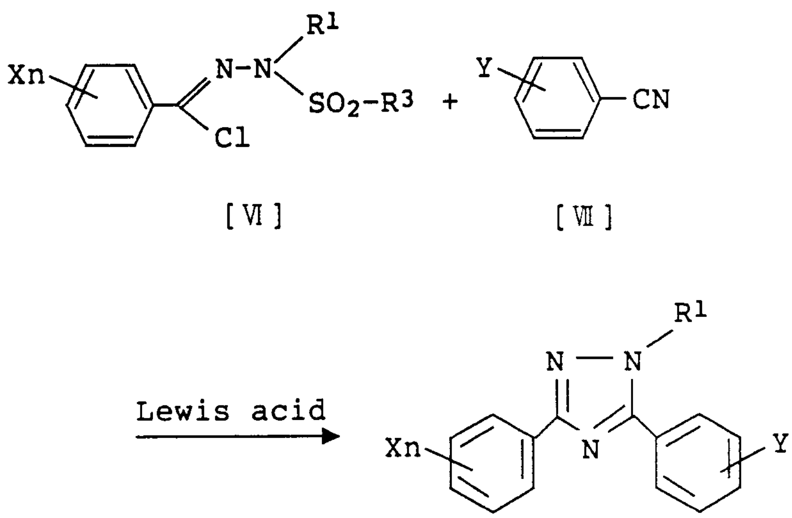


[VI] [VII]

[I]

(wherein $R^1$, X, n and Y have the same meaning as mentioned above, and $R^3$ is benzene or benzene substituted with an alkyl group having a carbon number of 1-4).

As the solvent, use may be made of any solvent not obstruction the reaction, which includes, for example, an ether such as diethyl ether, tetrahydrofuran, dioxane, diglyme or the like; an aromatic hydrocarbon such as benzene, toluene, chlorobenzene, dichlorobenzene or the like; an aliphatic hydrocarbon such as pentane, hexane, petroleum ether or the like; a halogenated hydrocarbon such as dichloromethane, dichloroethane, chloroform, carbon tetrachloride or the like; a non-protonic polar solvent such as nitrobenzene, N,N-dimethylformamide, N,N-dimethylacetamide, dimethylsulfoxide or the like; and a mixture thereof.

As the Lewis acid, use may be made of aluminum bromide, aluminium chloride, ferric chloride, boron trifluoride, titanium tetrachloride and the like.

In general, the amount of the compound of the general formula [VII] used is 1.0-2.0 moles per 1 mole of the compound of the general formula [VI], and the amount of Lewis acid used is 1.0-2.0 moles per 1 mole of the compound of the general formula [VI].

The reaction temperature is optionally within a range of 0°C to a boiling point of the solvent, but is preferably within a range of 50-180°C. The reaction time is dependent upon the kind of the compounds used, but is usually within a range of 15 minutes to 8 hours.

A concrete example of this reaction is disclosed, for example, in BULLETIN of the CHEMICAL SOCIETY of JAPAN, vol. 56, pages 545~548 (1983).

Production Method D

The compound of the general formula [I] according to the invention can be obtained by reacting an N-(phenylsulfonyl) benzamidrazone derivative of a general formula [VIII] with a benzoylhalide derivative of the general formula [V] in the absence of a solvent or in an inert solvent according to the following reaction formula (4):

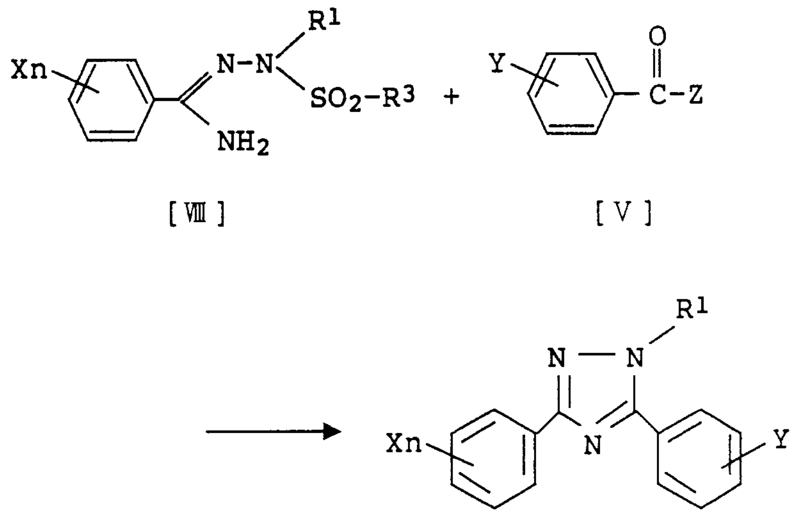

[VIII] [V]

[I]

(wherein $R^1$, $R^3$, X, n, Y and Z have the same meaning as mentioned above).

As the solvent, use may be made of any solvent not obstruction the reaction, which includes, for example, an ether such as diethyl ether, tetrahydrofuran, dioxane, diglyme or the like; an aromatic hydrocarbon such as benzene, toluene, chlorobenzene or the like; an aliphatic hydrocarbon such as pentane, hexane, petroleum ether or the like; a halogenated hydrocarbon such as dichloromethane, dichloroethane, chloroform, carbon tetrachloride or the like; an aprotic polar solvent such as N,N-dimethylformamide, N,N-dimethylacetamide, dimethylsulfoxide, 1-methyl-2-pyrolidinone or the like; and a mixture thereof.

In general, the amount of the compound of the general formula [V] used is 1.0-2.0 moles per 1 mole of the compound of the general formula [VIII].

The reaction temperature is optionally within a range of 0°C to a boiling point of the solvent, but is preferably within a range of 50-250°C. The reaction time is dependent upon the kind of the compounds used, but is usually within a range of 30 minutes to 5 hours.

A concrete example of this reaction is disclosed, for example, in Bulletin of the Chemical Society of Japan, vol. 56, page 548 (1983).

The compound of the general formula [VIII] as a starting material can be produced by the following method.

<u>Production Method E</u>

The compound of the general formula [VIII] can be obtained by reacting the compound of the general formula [VI] with ammonia gas in an inert solvent according to the following reaction formula (5):

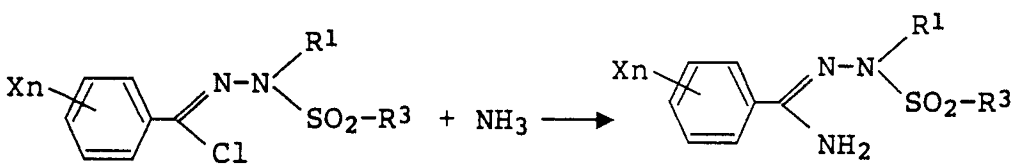

[VI] [VIII]

(wherein $R^1$, $R^3$, X and n have the same meaning as mentioned above).

As the solvent, use may be made of any solvent not obstruction the reaction, which includes, for example, an ether such as diethyl ether, tetrahydrofuran, dioxane, diglyme or the like; an aromatic hydrocarbon such as benzene, toluene, chlorobenzene or the like; an aliphatic hydrocarbon such as pentane, hexane, petroleum ether or the like; a halogenated hydrocarbon such as dichloromethane, dichloroethane, chloroform, carbon tetrachloride, diclorobenzene or the

like; an aprotic polar solvent such as N,N-dimethylformamide, N,N-dimethylacetamide, dimethylsulfoxide or the like; and a mixture thereof.

In general, the amount of ammonia gas used is 5.0-10.0 moles per 1 mole of the compound of the general formula [VI].

The reaction temperature is optionally within a range of 0°C to a boiling point of the solvent, but is preferably within a range of 20-150°C. The reaction time is dependent upon the kind of the compounds used, but is usually within a range of 1-24 hours.

A concrete example of this reaction is disclosed, for example, in BULLETIN of the CHEMICAL SOCIETY of JAPAN, vol. 56, pages 545~548 (1983).

The invention will be described concretely with reference to the following production examples, formulation examples and applications.

Production Example 1: 3-(2-chloro-6-fluorophenyl)-1-methyl-5-(4-octylphenyl)-1H-1,2,4-triazole (Compound No. 15)

In 100 ml of toluene were dissolved 2.20 g of ethyl 2-chloro-6-fluorobenzimidate and 1.10 g of triethylamine, to which was added dropwise 2.53 g of 4-octylbenzoyl chloride within a temperature range of 5-10°C with stirring and then stirred at room temperature for 1 hour and further refluxed under heating for 2 hours. After the cooling to room temperature, the resulting reaction solution was added with 100 ml of toluene, washed with a diluted hydrochloric acid and further with a saline solution, and thereafter the resulting toluene layer was dried over anhydrous magnesium sulfate.

The toluene layer was added with 3.00 g of monomethylhydrazine and stirred at room temperature for 8 hours. After the completion of the reaction, the reaction mixture was washed with a diluted hydrochloric acid solution and further with a saturated saline solution, dried over anhydrous magnesium sulfate and concentrated under a reduced pressure. The resulting concentrate was purified through a chromatography of silica gel column using a mixed solution of hexane and ethyl acetate as a developing solvent to obtain 1.34 g of the given compound ($n_D^{20}$ = 1.5652).

| NMR data (60 MHz, $CDCl_3$ solvent, δ value) | |
|---|---|
| 0.77 | (3H, t) |
| 1.00-1.79 | (12H, m) |
| 2.57 | (2H, t) |
| 3.95 | (3H, s) |
| 6.83-7.67 | (7H, m) |

Production Example 2: 3-(2-chlorophenyl)-1-methyl-5-[4-(6-methylhexyl)phenyl]-1H-1,2,4-triazole (Compound No. 67)

A mixture of 2.06 g of N-methyl-N-phenylsulfonyl-2-chlorobenzohydrazonoyl chloride, 1.30 g of 4-(6-methylhexyl) benzonitrile, 0.93 g of anhydrous aluminum chloride and 5 ml of o-dichlorobenzene was stirred in an oil bath at a temperature of 140°C for 30 minutes. After the cooling, the resulting solution was dissolved in 200 ml of chloroform, washed with diluted hydrochloric acid solution, diluted sodium hydroxide aqueous solution and saline water in this order, dried over anhydrous magnesium sulfate and concentrated under a reduced pressure. The resulting concentrate was purified through a chromatography of silica gel column using a mixed solution of hexane and ethyl acetate as a developing solvent to obtain 1.52 g of the given compound (melting point: 64.0-67.0°C).

| NMR data (60 MHz, $CDCl_3$ solvent, δ value) | |
|---|---|
| 0.86 | (6H, d) |
| 1.15-1.80 | (7H, m) |
| 2.67 | (2H, t) |
| 4.00 | (3H, s) |
| 7.17-8.00 | (8H, m) |

Production Example 3: 3-(2-chlorophenyl)-1-methyl-5-(4-tridecylphenyl)-1H-1,2,4-triazole (Compound No. 42)

A mixture of 0.82 g of N-methyl-N-phenylsulfonyl-2-chlorobenzohydrazonoyl chloride, 0.70 g of 4-tridecylbenzonitrile, 0.4 g of anhydrous aluminium chloride and 3 ml of o-dichlorobenzene was stirred in an oil bath at a temperature of 140°C for 30 minutes. After the cooling, the resulting solution was dissolved in 100 ml of chloroform, washed with diluted hydrochloric acid solution, diluted sodium hydroxide solution and saline water in this order, dried over anhydrous magnesium sulfate and concentrated under a reduced pressure. The resulting concentrate was purified through a chromatography of silica gel column using a mixed solution of hexane and ethyl acetate as a developing solvent to obtain 0.70 g of the given compound (melting point: 55.0-57.0°C).

| NMR data (60 MHz, $CDCl_3$ solvent, δ value) | |
|---|---|
| 0.67-1.80 | (25H, m) |
| 2.67 | (2H, t) |
| 4.00 | (3H, s) |
| 7.16-8.03 | (8H, m) |

Production Example 4: 3-(2-chlorophenyl)-1-methyl-5-(4-pentadecylphenyl)-1H-1,2,4-triazole (Compound No. 50)

A mixture of 3.24 g of N-methyl-N-phenylsulfonyl-2-chlorobenzamidrazone and 3.50 g of 4-pentadecylbenzoyl chloride was stirred in an oil bath at a temperature of 170-180°C for 4 hours. After the cooling, the resulting solution was added with water and extracted with ethyl acetate (200 ml x 2) and the extracted organic layer was washed with saline water, dried over anhydrous magnesium sulfate and concentrated under a reduced pressure. The resulting concentrate was purified through a chromatography of silica gel column using a mixed solution of hexane and ethyl acetate as a developing solvent and washed with n-hexane to obtain 0.34 g of the given compound (melting point: 62.0-65.0°C).

| NMR data (60 MHz, $CDCl_3$ solvent, δ value) | |
|---|---|
| 0.77-1.73 | (29H, m) |
| 1.67 | (2H, m) |
| 4.00 | (3H, s) |
| 7.17-7.97 | (8H, m) |

Production Example 5: 5-(4-decyloxyphenyl)-3-(2,6-dichlorophenyl)-1-methyl-1H-1,2,4-triazole (Compound No. 85)

A mixture of 1.10 g of N-methyl-N-phenylsulfonyl-2,6-dichlorobenzohydrazonoyl chloride, 0.70 g of 4-decyloxybenzonitrile, 0.4 g of anhydrous aluminium chloride and 3 ml of o-dichlorobenzene was stirred in an oil bath at a temperature of 140°C for 30 minutes. After the cooling, the resulting solution was dissolved in 100 ml of chloroform, washed with diluted hydrochloric acid solution, diluted sodium hydroxide solution and saline water in this order, dried over anhydrous magnesium sulfate and concentrated under a reduced pressure. The resulting concentrate was purified through a chromatography of silica gel column using a mixed solution of hexane and ethyl acetate as a developing solvent to obtain 0.40 g of the given compound (melting point: 60.0-64.0°C).

| NMR data (60 MHz, $CDCl_3$ solvent, δ value) | |
|---|---|
| 0.77-1.90 | (19H, m) |
| 3.98 | (2H, t) |
| 4.04 | (3H, s) |
| 6.88-7.73 | (7H, m) |

Production Example 6: 3-(2-chloro-6-fluorophenyl)-5-[4-(3-chloro-5-trifluoromethylpyridin-2-yloxy)phenyl]-1-methyl-1H-1,2,4-triazole (Compound No. 429)

A mixture of 1.30 g of N-methyl-N-phenylsulfonyl-2-chloro-6-fluorobenzohydrazonoyl chloride, 1.00 g of 4-(3-chloro-5-trifluoromethylpyridin-2-yloxy)-benzonitrile, 0.50 g of anhydrous aluminum chloride and 3 ml of o-dichlorobenzene was stirred in an oil bath at a temperature of 140°C for 30 minutes. After the cooling, the resulting solution was dissolved in 100 ml of chloroform, washed with diluted hydrochloric acid solution, diluted sodium hydroxide solution and saline water in this order, dried over anhydrous magnesium sulfate and concentrated under a reduced pressure. The resulting concentrate was purified through a chromatography of silica gel column using a mixed solution of hexane and ethyl acetate as a developing solvent to obtain 0.70 g of the given compound (measurement of $n_D^{20}$ was impossible).

| NMR data (60 MHz, $CDCl_3$ solvent, δ value) | |
|---|---|
| 4.07 | (3H, s) |
| 6.75-8.58 | (9H, m) |

Production Example 7: N-methyl-N-phenylsulfonyl-2-chlorobenzamidrazone

In 100 ml of N,N-dimethylformamide was dissolved 17.2 g of N-methyl-N-phenylsulfonyl-2-chlorobenzhydrazonoyl chloride, which was stirred at 60-70°C for 3 hours while introducing ammonia gas thereinto. After the cooling, the reaction solution was dissolved in 500 ml of ethyl acetate, washed with water, dried on anhydrous magnesium sulfate and concentrated under a reduced pressure. The resulting crystal was washed with n-hexane to obtain 15.4 g of the given compound (melting point: 94.0-96.0°C).

| NMR data (60 MHz, $CDCl_3$ solvent, δ value) | |
|---|---|
| 2.75 | (3H, s) |
| 5.80 | (2H, s) |
| 7.10-8.00 | (9H, m) |

The insecticide and acaricide according to the invention contain the triazole derivative represented by the general formula (I) as an active ingredient.

When the triazole compounds according to the invention are used as an active ingredient for insecticides and acaricides, these compounds themselves may be used alone, or may be compounded with a carrier, a surfactant, a dispersing agent, an adjuvant or the like usually used in the formulation to form dusts, wettable powder, emulsion, fine powder, granulates or the like.

As the carrier used in the formulation, mention may be made of a solid carrier such as zeeklite, talc, bentonite, clay, kaolin, diatomaceous earth, white carbon, vermiculite, calcium hydroxide, quartz sand, ammonium sulfate, urea or the like; and a liquid carrier such as isopropyl alcohol, xylene, cyclohexane, methylnaphthalene or the like.

As the surfactant and dispersing agent, mention may be made of a metal salt of alkylbenzene sulfonic acid, a metal salt of dinaphtylmethane disulfonic acid, a sulfuric acid ester of alcohol, alkylarylsulfonate, lignin sulfonate, polyoxyethylene glycol ether, polyoxyethylene alkylaryl ether, polyoxyethylene sorbitan monoalkylate and the like.

As the adjuvant, mention may be made of carboxymethylcellulose, polyethylene glycol, gum arabi and the like.

In use, the compound according to the invention is directly applied or sprayed by diluting to a proper concentration.

The insecticide and acaricide according to the invention may be used by spraying onto stem and leaves, by applying to soil, by applying to a nursery box, by spraying onto water surface or the like.

In the formulation, the amount of the active ingredient used may be selected in accordance with the use purpose, but it is properly selected within a range of 0.05-20% by weight, preferably 0.1-10% by weight in case of the dusts or granules. In case of the emulsion or wettable powder, the amount of the active ingredient is properly selected within a range of 0.5-80% by weight, preferably 1-60% by weight.

The amount of the insecticide and acaricide applied is dependent upon the kind of the compound used as an active ingredient, injurious insect to be controlled, tendency and degree of insect injury, environmental condition, kind of formulation used and the like. When the insecticide and acaricide according to the invention are directly used as dusts or granules, the amount of the active ingredient is properly selected within a range of 0.05 g - 5 kg, preferably 0.1-1 kg per 10 are. Furthermore, when they are used in form of a liquid as emulsion or wettable powder, the amount of the active ingredient is properly selected within a range of 0.1-5000 ppm, preferably 1-1000 ppm.

Moreover, the insecticide and acaricide according to the invention may be used by mixing with other insecticide, fungicide, fertilizer, plant growth regulator and the like.

The formulation will concretely be described with respect to typical examples. In this case, the kind of the compounds and additives and the compounding ratio are not limited to these examples and may be varied within wide ranges. Moreover, % is by weight otherwise specified.

Formulation Example 1: Emulsion

An emulsion was prepared by uniformly dissolving 30% of compound No. 55, 20% of cyclohexanone, 11% of polyoxyethylene alkylaryl ether, 4% of calcium alkylbenzenesulfonate and 35% of methylnaphthalene.

Formulation Example 2: Wettable powder

A wettable powder was prepared by uniformly mixing and pulverizing 40% of compound No. 38, 15% of diatomaceous earth, 15% of clay, 25% of white carbon, 2% of sodium dinaphthylmethane disulfonate and 3% of sodium lignin sulfonate.

Formulation Example 3: Dust

A dust was prepared by uniformly mixing and pulverizing 2% of compound No. 120, 5% of diatomaceous earth and 93% of clay.

Formulation Example 4: Granules

A mixture of 5% of compound No. 71, 2% of sodium salt of lauryl alcohol sulfuric acid ester, 5% of sodium lignin sulfonate, 2% of carboxymethyl cellulose and 86% of clay was uniformly pulverized and added with 20 parts by kneaded, shaped into granules of 14-32 mesh through an extrusion type granulating machine and dried to form granules.

The triazole derivatives according to the invention are effective to control planthoppers such as brown planthopper, white-backed planthopper, small brown planthopper and the like; leafhoppers such as green rice leafhopper, tea green leafhopper and the like; aphids such as cotton aphid, green peach aphid, cabbage aphid and the like; whiteflies such as greenhouse whitefly and the like; hemipteran injurious insects such as mulberry scale, corbett rice bug and the like; lepidopteran injurious insects such as diamond-back moth, lima-bean cutworm, tobacco cutworm and the like; dipteran

injurious insects such as house maggot, mosquito and the like; elytron injurious insects such as rice plant weevil, soy bean weevil, cucurbit leaf beetle and the like; orthopteran injurious insects such as american cockroach, steam fly and the like; mites such as two-spotted spider mite, kanzawa spider mite, citrus red mite and the like; and mites having an increased resistance to organotin, synthesized pyrethroid and organophosphorus chemicals.

Particularly, they develop a very excellent effect of controlling mites such as two-spotted spider mite, kanzawa spider mite, citrus red mite and the like.

The effect of the compounds according to the invention will be described with respect to the following test examples. Moreover, the following compounds were used as a comparative chemical, wherein a comparative chemical a is a compound described in Japanese Patent laid open No. 56-154464, and a comparative chemical b is a commercial product usually used for the control of mites.

Comparative chemical A: 3,5-bis(o-chlorophenyl)-1-methyl-1H-1,2,4-triazole

Comparative chemical B: Hexythiazox (common name)

Test Example 1: Insecticidal test for diamond-back moth

The wettable powder prepared according to Formulation Example 2 was diluted with water so that the concentration of the active ingredient was 500 ppm. Cabbage leaves were immersed in the resulting diluted solution, dried in air and then placed in a vinyl chloride cup of 60 ml capacity. Ten larvae of 3rd instar diamondback moth were released in the cup and thereafter a cover was placed thereon. Then, the cup was placed in a thermostatic chamber of 25°C for 6 days, and the number of larvae died was counted to calculate the percentage of mortality. The test was carried out by double series. Moreover, the comparative chemical A was used for the comparison. The results are shown in Table 26.

Table 26

| Compound No. | Mortality (%) | Compound No. | Mortality (%) |
|---|---|---|---|
| 17 | 90 | 479 | 100 |
| 30 | 100 | 483 | 100 |
| 37 | 90 | 494 | 100 |
| 43 | 95 | 500 | 100 |
| 71 | 95 | 504 | 95 |
| 120 | 95 | 510 | 100 |
| 135 | 90 | 514 | 100 |
| 140 | 95 | 520 | 100 |
| 185 | 100 | 524 | 90 |
| 207 | 90 | 526 | 100 |
| 217 | 100 | 528 | 100 |
| 240 | 100 | 530 | 100 |
| 244 | 100 | 532 | 100 |
| 427 | 100 | Comparative chemical A | 20 |
| 471 | 95 | | |

Test Example 2: Insecticidal test for larvae of cotton aphid

The wettable powder prepared according to Formulation Example 2 was diluted with water so that the concentration of the active ingredient was 100 ppm. In the resulting diluted solution were immersed cucumber seedlings previously inoculated with larvae of cotton aphid and then subjected to a drying treatment in air. After the treatment, the cucumber seedlings were placed in a thermostatic chamber of 25°C for 3 days and then the number of larvae died was counted to calculate the percentage of mortality. The test was carried out by double series. The results are shown in Table 27.

Table 27

| Compound No. | Mortality (%) | Compound No. | Mortality (%) | Compound No. | Mortality (%) |
|---|---|---|---|---|---|
| 3 | 100 | 109 | 100 | 229 | 100 |
| 7 | 100 | 111 | 100 | 234 | 100 |
| 13 | 100 | 112 | 100 | 235 | 100 |
| 14 | 100 | 113 | 100 | 239 | 100 |
| 15 | 100 | 114 | 100 | 240 | 100 |
| 17 | 100 | 117 | 100 | 247 | 100 |
| 19 | 100 | 118 | 100 | 425 | 100 |
| 30 | 100 | 119 | 100 | 427 | 100 |
| 35 | 100 | 127 | 100 | 434 | 100 |
| 39 | 100 | 131 | 100 | 435 | 100 |
| 47 | 100 | 135 | 100 | 447 | 100 |
| 51 | 100 | 140 | 100 | 450 | 100 |
| 68 | 100 | 144 | 100 | 451 | 100 |
| 69 | 100 | 146 | 100 | 452 | 100 |
| 71 | 100 | 148 | 100 | 468 | 100 |
| 73 | 100 | 149 | 100 | 469 | 100 |
| 81 | 100 | 150 | 100 | 475 | 100 |
| 84 | 100 | 151 | 100 | 490 | 100 |
| 87 | 100 | 152 | 100 | 494 | 100 |
| 89 | 100 | 153 | 100 | 503 | 100 |
| 96 | 100 | 163 | 100 | 504 | 100 |
| 97 | 100 | 190 | 100 | 510 | 100 |
| 100 | 100 | 194 | 100 | 516 | 100 |
| 101 | 100 | 195 | 100 | 518 | 100 |
| 103 | 100 | 220 | 100 | 520 | 100 |
| 104 | 100 | 223 | 100 | 522 | 100 |
| 105 | 100 | 224 | 100 | 528 | 100 |
| 106 | 100 | 225 | 100 | 530 | 100 |
| 107 | 100 | 228 | 100 | 532 | 100 |
| 108 | 100 | | | | |

Test Example 3: Ovicidal test for eggs of two-spotted spider mite

Female adults of two-spotted spider mite were placed on three leaf discs of kidney bean (diameter: 15 mm) and oviposited over 24 hours, and thereafter these adults were removed therefrom. The wettable powder prepared according to Formulation Example 2 was diluted with water so that the concentration of the active ingredient was 0.16 ppm. In the resulting diluted solution were immersed these leaf discs for 10 seconds. After the treatment, the leaf discs were placed in a thermostatic chamber of 25°C for 7 days and then the number of unhatched eggs was counted to calculate the percentage of ovicidal activity. The test was carried out by double series. Moreover, the comparative chemicals A and B were used for the comparison. The results are shown in Table 28.

Table 28

| Compound No. | Ovicidal activity (%) |
|---|---|
| 21 | 100 |
| 30 | 100 |
| 34 | 100 |
| 35 | 100 |
| 38 | 100 |
| 39 | 95 |
| 42 | 100 |
| 43 | 95 |
| 47 | 100 |
| 50 | 100 |
| 51 | 100 |
| 54 | 100 |
| 55 | 100 |
| Comparative chemical A | 24 |
| Comparative chemical B | 95 |

Test Example 4: Ovicidal test for eggs of chemical-resistant kanzawa spider mite

Female adults of kanzawa spider mite having a resistance to commercially available chemicals were placed on three leaf discs of kidney bean (diameter: 15 mm) and oviposited over 2 days, and thereafter these adults were removed therefrom. The wettable powder prepared according to Formulation Example 2 was diluted with water so that the concentration of the active ingredient was 4 ppm. In the resulting diluted solution were immersed these leaf discs for 10 seconds. After the treatment, the leaf discs were placed in a thermostatic chamber of 25°C for 7 days and then the number of unhatched eggs was counted to calculate the percentage of ovicidal activity. The test was carried out by double series. Moreover, the comparative chemicals A and B were used for the comparison. The results are shown in Table 29 and Table 30.

Table 29

| Compound No. | Ovicidal activity (%) | Compound No. | Ovicidal activity (%) |
|---|---|---|---|
| 3 | 100 | 81 | 100 |
| 6 | 100 | 83 | 100 |
| 13 | 100 | 84 | 90 |
| 14 | 100 | 88 | 100 |
| 15 | 100 | 89 | 100 |
| 30 | 100 | 106 | 100 |
| 33 | 100 | 110 | 100 |
| 34 | 100 | 111 | 95 |
| 35 | 100 | 112 | 100 |
| 36 | 100 | 117 | 100 |
| 37 | 100 | 118 | 100 |
| 38 | 100 | 140 | 100 |
| 39 | 100 | 148 | 100 |
| 40 | 100 | 151 | 100 |
| 41 | 100 | 153 | 100 |
| 42 | 100 | 166 | 100 |
| 43 | 100 | 167 | 100 |
| 44 | 100 | 168 | 100 |
| 46 | 90 | 183 | 100 |
| 47 | 100 | 191 | 100 |
| 48 | 100 | 192 | 100 |
| 51 | 100 | 193 | 100 |
| 52 | 95 | 204 | 100 |
| 53 | 90 | 205 | 100 |
| 55 | 100 | 206 | 100 |
| 56 | 100 | 217 | 100 |
| 57 | 90 | 223 | 100 |

Table 30

| Compound No. | Ovicidal activity (%) | Compound No. | Ovicidal activity (%) |
|---|---|---|---|
| 225 | 100 | 473 | 100 |
| 232 | 100 | 475 | 100 |
| 235 | 100 | 477 | 100 |
| 239 | 100 | 479 | 100 |
| 240 | 100 | 481 | 100 |
| 247 | 100 | 483 | 100 |
| 425 | 100 | 487 | 100 |
| 426 | 100 | 489 | 100 |
| 427 | 100 | 494 | 100 |
| 428 | 100 | 498 | 100 |
| 429 | 100 | 500 | 100 |
| 433 | 100 | 501 | 100 |
| 434 | 100 | 503 | 100 |
| 437 | 100 | 504 | 100 |
| 444 | 100 | 510 | 100 |
| 445 | 100 | 514 | 100 |
| 447 | 100 | 516 | 100 |
| 450 | 100 | 518 | 100 |
| 451 | 100 | 520 | 100 |
| 452 | 100 | 522 | 100 |
| 454 | 100 | 524 | 100 |
| 461 | 100 | 526 | 100 |
| 465 | 100 | 530 | 100 |
| 466 | 100 | 532 | 100 |
| 468 | 100 | Comparative chemical A | 31 |
| 469 | 100 | | |
| 471 | 100 | Comparative chemical B | 0 |

Test Example 5: Insecticidal test for larvae of chemical-resistant kanzawa spider mite

Female adults of kanzawa spider mite having a resistance to commercially available chemicals were placed on three leaf discs of kidney bean (diameter: 15 mm) and oviposited over 2 days, and thereafter these adults were removed therefrom. Then, these leaf discs were placed in a thermostatic chamber of 25°C for 5 days and the number of hatched larvae was counted. Separately, the wettable powder prepared according to Formulation Example 2 was diluted with water so that the concentration of the active ingredient was 20 ppm. After these leaf discs were sprayed with the resulting diluted solution, they were placed in a thermostatic chamber of 25°C for 7 days and then the number of living adults was counted to calculate the percentage of mortality on the hatched larvae. The test was carried out by double series. Moreover, the comparative chemicals A and B were used for the comparison. The results are shown in Table 31.

Table 31

| Compound No. | Mortality (%) | Compound No. | Mortality (%) |
|---|---|---|---|
| 3 | 100 | 41 | 100 |
| 13 | 100 | 42 | 100 |
| 14 | 100 | 43 | 100 |
| 15 | 100 | 44 | 100 |
| 16 | 100 | 45 | 100 |
| 17 | 100 | 46 | 100 |
| 18 | 100 | 47 | 100 |
| 21 | 100 | 48 | 100 |
| 30 | 100 | 49 | 100 |
| 31 | 100 | 50 | 100 |
| 32 | 100 | 51 | 100 |
| 34 | 100 | 52 | 100 |
| 35 | 100 | 53 | 100 |
| 36 | 100 | 55 | 100 |
| 37 | 100 | 56 | 100 |
| 38 | 100 | Comparative chemical A | 55 |
| 39 | 100 | Comparative chemical B | 25 |
| 40 | 100 | | |

Test Example 6: Ovicidal test for eggs of citrus red mite

Female adults of citrus red mite were placed on two laminae of citrus fruit (diameter: 10 mm) and oviposited over 2 days, and thereafter these adults were removed therefrom. The wettable powder prepared according to Formulation Example 2 was diluted with water so that the concentration of the active ingredient was 4 ppm. In the resulting diluted solution were immersed these laminae for 10 seconds. After the treatment, the laminae were placed in a thermostatic chamber of 25°C for 7 days and then the number of unhatched eggs was counted to calculate the percentage of ovicidal activity. The test was carried out by double series. Moreover, the comparative chemicals A and B were used for the comparison. The results are shown in Table 32.

Table 32

| Compound No. | Ovicidal activity (%) |
|---|---|
| 3 | 95 |
| 16 | 90 |
| 17 | 100 |
| 18 | 100 |
| 21 | 95 |
| 30 | 100 |
| 31 | 100 |
| 32 | 100 |
| 34 | 100 |
| 35 | 100 |
| 36 | 95 |
| 37 | 100 |
| 38 | 100 |
| 39 | 100 |
| 40 | 90 |
| 43 | 100 |
| 44 | 100 |
| 47 | 95 |
| 48 | 100 |
| 52 | 100 |
| 97 | 95 |
| 106 | 100 |
| Comparative chemical A | 33 |
| Comparative chemical B | 90 |

## Claims

**1.** A triazole derivative having the following general formula [I]:

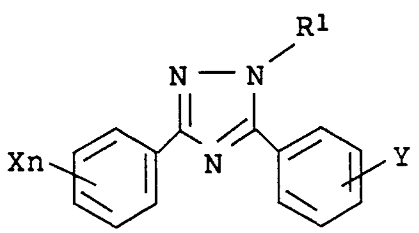

[I]

[wherein $R^1$ is an alkyl group, X is a hydrogen atom, a halogen atom, an alkyl group, an alkoxy group, an alkylthio group, a nitro group, a cyano group or trifluoromethyl group, n is an integer of 1-5 provided that when n is 2 or more,

X may be an optional combination of same or different atoms or groups, and Y is an alkenyl group, an alkynyl group, an alkoxyalkyl group, an alkoxyalkoxy group, an alkylthioalkyl group, a cycloalkyl group, a cycloalkylalkoxy group, a cycloalkylalkyl group, a cycloalkylalkenyl group, a cycloalkylalkynyl group, a trialkylsilylalkyl group, a trialkylsilylalkoxy group, an alkyl group having a carbon number of not less than 7, an alkoxy group having a carbon number of not less than 7, an alkylthio group having a carbon number of not less than 7, an alkylsulfinyl group having a carbon number of not less than 7, an alkylsulfonyl group having a carbon number of not less than 7 or a group represented by the following general formula (1):

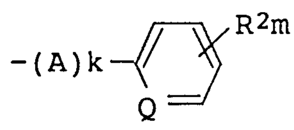

(wherein A is an oxygen atom, a sulfur atom, a lower $C_1$-$C_4$ alkylene group, a lower $C_1$-$C_4$ alkyleneoxy group, an oxy-lower $C_1$-$C_4$ alkylene group or a lower $C_2$-$C_8$ alkyleneoxyalkylene group, k is 0 or 1, Q is CH- group or a nitrogen atom, $R^2$ is a hydrogen atom, a halogen atom, an alkyl group, an alkoxy group, trifluoromethyl group or trifluoromethoxy group, m is an integer of 1-5 provided that when m is 2 or more, $R^2$ may be an optional combination of same or different atoms or groups)].

2. A triazole derivative according to claim 1, wherein said $R^1$ is a straight or branched-chain alkyl group having a carbon number of 1-6, X is a hydrogen atom, a halogen atom, a straight or branched-chain alkyl group having a carbon number of 1-4, a nitro group, a cyano group or trifluoromethyl group, n is an integer of 1-3 provided that when n is 2 or 3, X may be an optional combination of same or different atoms or groups, Y is a straight or branched-chain alkyl group having a carbon number of 7-20, a cycloalkyl group having a carbon number of 3-12, a cycloalkylalkyl group having a carbon number of 6-12, a straight or branched-chain alkoxy group having a carbon number of 7-16, a cycloalkylalkoxy group having carbon number of 7-12, a straight or branched-chain alkylthio group having a carbon number of 7-16, an alkylsulfinyl group, an alkylsulfonyl group, a straight or branched-chain alkenyl group having a carbon number of 3-16, a cycloalkylalkenyl group having a carbon number of 5-12, a straight or branched-chain alkynyl group having a carbon number of 3-16, a cycloalkylalkynyl group having a carbon number of 5-12, a tri(lower $C_1$-$C_6$ alkyl)silyl lower $C_1$-$C_6$ alkyl group, a tri(lower $C_1$-$C_6$ alkyl)silyl lower $C_1$-$C_6$ alkoxy group or a group represented by said formula (1) (wherein A is an oxygen atom, a sulfur atom, a lower alkylene group having a carbon number of 1-4, methyleneoxy group or oxymethylene group, k is 0 or 1, Q is CH- group or a nitrogen atom, $R^2$ is a hydrogen atom, a halogen atom, a lower $C_1$-$C_6$ alkyl group, a lower $C_1$-$C_6$ alkoxy group, trifluoromethyl group or trifluoromethoxy group, and m is an integer of 1-3 provided that when m is 2 or 3, $R^2$ may be an optional combination of same or different atoms or groups).

3. A triazole derivative according to claim 2, wherein said $R^1$ is methyl group.

4. An insecticide containing a triazole derivative claimed in claim 1 as an active ingredient.

5. An acaricide containing a triazole derivative claimed in claim 1 as an active ingredient.

6. A method of producing a triazole derivative having the following general formula [I]:

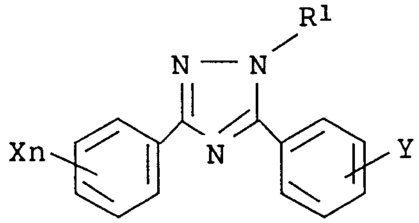

[ I ]

(wherein $R^1$, X, Y and n have the same meaning as in claim 1) which comprises reacting a compound represented by the following general formula [II]:

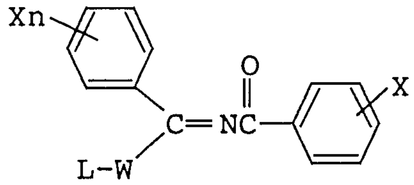

[ II ]

(wherein W is a sulfur atom or an oxygen atom, L is an alkyl group having a carbon number of 1-4 and X, n and Y have the same meaning as mentioned above) with a hydrazine derivative represented by a general formula [III] of $R^1NHNH_2$ (wherein $R^1$ has the same meaning as mentioned above).

**7.** A method of producing a triazole derivative having the following general formula [I]:

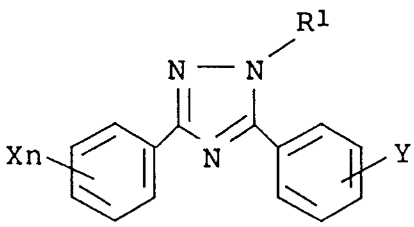

[ I ]

(wherein $R^1$, X, Y and n have the same meaning as in claim 1) which comprises reacting a compound represented by the following general formula [VI]:

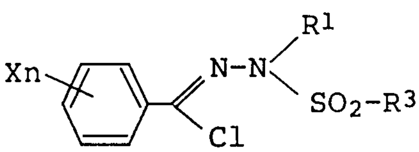

[ VI ]

(wherein $R^1$, X and n have the same meaning as mentioned above, and $R^3$ is benzene or benzene substituted with an alkyl group having a carbon number of 1-4) with a benzonitrile derivative represented by the following general formula [VII]:

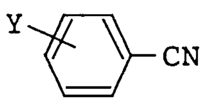

[ VII ]

(wherein Y has the same meaning as mentioned above) in the presence of Lewis acid.

**8.** A method of producing a triazole derivative having the following general formula [I]:

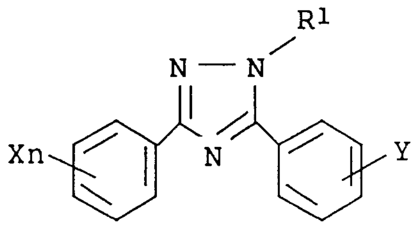

[ I ]

(wherein $R^1$, X, Y and n have the same meaning as in claim 1) which comprises reacting a compound represented by the following general formula [VIII]:

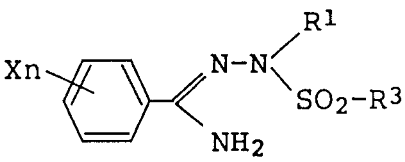

[ VIII ]

(wherein $R^1$, X and n have the same meaning as mentioned above, and $R^3$ is benzene or benzene substituted with an alkyl group having a carbon number of 1-4) with a compound represented by the following general formula [V]:

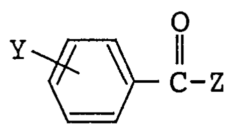

[ V ]

(wherein Z is a halogen atom and Y has the same meaning as mentioned above).

**Patentansprüche**

**1.** Triazole-Derivat mit der folgenden allgemeinen Formel [I]:

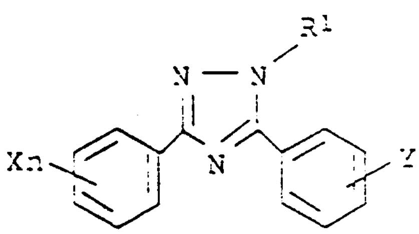

[I]

[wobei $R^1$ eine Alkylgruppe ist, X ein Wasserstoffatom, ein Halogenatom, eine Alkylgruppe, eine Alkoxygruppe, eine Alkylthiogruppe, eine Nitrogruppe, eine Cyangruppe oder eine Trifluormethylgruppe bedeutet, n eine ganze Zahl von 1-5 ist, und sofern n = 2 oder mehr ist, kann X eine wahlweise Kombination von gleichen oder unterschiedlichen Atomen oder Gruppen sein, und Y eine Alkenylgruppe, eine Aklkynylgruppe, eine Alkoxyalkylgruppe, eine Alkoxyalkoxgruppe, eine Alkylthioalkylgruppe, eine Zycloalkylgruppe, eine Zycloalkylalkoxygruppe, eine Zycloalkylalkylgruppe, eine Zycloalkylalkenylgruppe, eine Zykloalkylalkynylgruppe, eine Trialkylsilylalkylgruppe, eine Trialkylsilylalkoxygruppe, eine Alkylgruppe mit einer Kohlenstoffzahl nicht geringer als 7, eine Alkoxygruppe mit einer Kohlenstoffzahl nicht geringer als 7, eine Alkylthiogruppe mit einer Kohlenstoffzahl nicht geringer als 7, eine Alkylsulfinylgruppe mit einer Kohlenstoffzahl nicht geringer als 7, eine Alkylsulfonylgruppe mit einer Kohlenstoffzahl nicht geringer als 7, oder eine Gruppe, welche durch die folgende allgemeine Formel wiedergegeben wird (1):

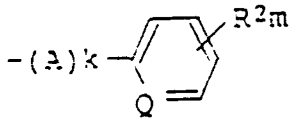

(worin A ein Sauerstoffatom, ein Schwefelatom, eine niedrige $C_1$ - $C_4$ Alkylengruppe, eine niedrige $C_1$ - $C_4$ Alkylenoxygruppe, eine oxy-niedrige $C_1$ - $C_4$ Alkylengruppe oder eine niedrige $C_2$ - $C_8$ Alkylenoxyalkylengruppe, k bedeutet 0 oder 1, Q ist eine CH-Gruppe oder ein Stickstoffatom, $R^2$ ist ein Wasserstoffatom, ein Halogenatom, eine Alkylgruppe, eine Alkoxygruppe, Trifluormethylgruppe oder Trifluormethoxygruppe, m ist eine ganze Zahl von 1 bis 5, und wenn m = 2 oder mehr ist, kann $R^2$ eine wahlweise Kombination von gleichen oder unterschiedlichen Atomen oder Gruppen sein)].

2. Triazole-Derivat nach Anspruch 1, wobei $R^1$ eine gerade oder verzweigtkettige Alkylgruppe mit einer Kohlenstoffzahl von 1-6 darstellt, X ist ein Wasserstoffatom, ein Halogenatom, eine gerade oder verzweigtkettige Alkylgruppe mit einer Kohlenstoffzahl von 1-4, eine Nitrogruppe, eine Zyan- oder Trifluormethylgruppe, n ist eine ganze Zahl von 1-3, und wenn n=2 oder 3 ist, kann X eine wahlweise Kombination von gleichen oder unterschiedlichen Atomen oder Gruppen sein, Y ist eine gerade oder verzweigtkettige Alkylgruppe mit einer Kohlenstoffzahl von 3-12, ein Zykloalkylalkylgruppe mit einer Kohlenstoffzahl von 6-12, eine gerade oder verzweigtkettige Alkoxygruppe mit einer Kohlenstoffzahl von 7-16, eine Zykloalkylalkoxygruppe mit einer Kohlenstoffzahl von 7-12, eine gerade oder verzweigtkettige Alkylthiogruppe mit einer Kohlenstoffzahl von von 7-16, eine Alkylsulfinylgruppe, eine Alkylsulfonylgruppe, eine gerade oder verzweigtkettige Alkenylgruppe mit einer Kohlenstoffzahl von 3-16, eine Zykloalkylalkenylgruppe mit einer Kohlenstoffzahl von 5-12, eine gerade oder verzweigtekettige Alkynylgruppe mit einer Kohlenstoffzahl von 3-16, eine Zykloalkylalkynylgruppe mit einer Kohlenstoffzahl von 5-12, eine Tri(untere $C_1$-$C_6$ Alkyl)Silyl untere $C_1$-$C_6$ Alkylgruppe, eine Tri(untere $C_1$-$C_6$ Alkyl)Silyl untere $C_1$-$C_6$ Alkoxygruppe oder eine Gruppe, welche durch die Formel (1) dargestellt wird, (worin A ein Sauerstoffatom, ein Schwefelatom, eine niedrige Alkylengruppe mit einer Kohlenstoffzahl von 1-4, Methylenoxygruppe oder Oxymethylengruppe darstellt, k ist 0 oder 1, Q ist eine CH-Gruppe oder ein Stickstoffatom, $R^2$ ein Wasserstoffatom, ein Halogenatom, eine niedrige $C_1$-$C_6$ Alkylgruppe, eine niedrige $C_1$-$C_6$ Alkoxygruppe, Trifluormethylgruppe oder Trifluormethoxygruppe, und m eine ganze Zahl von 1-3 ist, und falls m=2 oder 3 ist, kann $R^2$ eine wahlweise Kombination von gleichen oder unterschiedlichen Atomen oder Gruppen sein).

3. Triazole-Derivat nach Anspruch 2, wobei $R^1$ eine Methylgruppe ist.

4. Ein Insektizid enthaltend ein Triazol-Derivat nach Anspruch 1 als einen aktiven Bestandteil.

5. Ein Akarizid enthaltend ein Triazol-Derivat nach Anspruch 1 als einen aktiven Bestandteil.

6. Verfahren zur Herstellung eines Triazol-Derivats nach der folgenden allgemeinen Formel [I]:

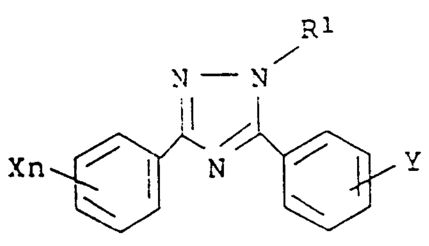

[I]

(worin $R^1$, X, Y und n die gleiche Bedeutung wie im Anspruch 1 haben), welches die Reaktion einer Verbindung aufweist, die durch die allgemeine Formel [II] dargestellt wird:

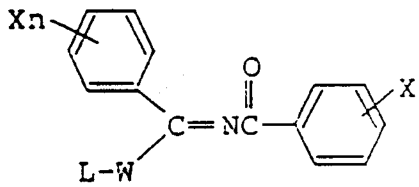

[ II ]

(wobei W ein Schwefelatom oder ein Sauerstoffatom darstellt, L eine Alkylgruppe mit einer Kohlenstoffzahl von 1-4 und X, n und Y die gleiche Bedeutung haben wie oben aufgeführt) mit einem Hydrazinderivat, welches durch die allgemeine Formel [III] $R^1NHNH^2$ dargestellt wird, (wobei $R^1$ die gleiche Bedeutung hat wie oben aufgeführt.

**7.** Verfahren zur Herstellung eines Triazole-Derivats mit der folgenden allgemeinen Formel [I]:

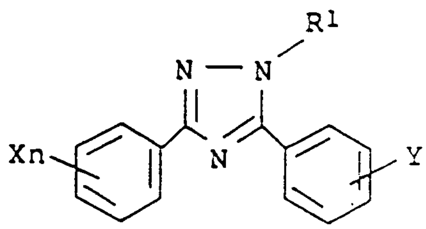

[I]

(wobei $R^1$, X, Y und n die gleiche Bedeutung wie im Anspruch 1 haben, welches die Reaktion einer Verbindung aufweist, die durch die folgende allgemeine Formel [VI] dargestellt wird:

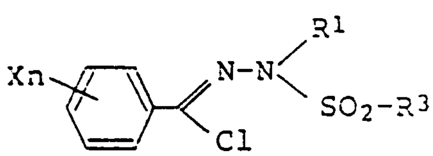

[VI]

(wobei $R^1$, X, Y und n die gleiche Bedeutung wie oben aufgeführt haben, und $R^3$ Benzol oder Benzol, substituiert mit einer Alkylgruppe einer Kohlenstoffzahl von 1-4) mit einem Benzonitrilderivat, dargestellt durch die folgende allgemeine Formel [VII]:

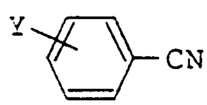

[VII]

(worin Y die gleiche Bedeutung wie oben aufgeführt hat) unter Anwesenheit von Lewis-Säure.

8. Verfahren zur Herstellung eines Triazole-Derivats mit der folgenden allgemeinen Formel [I]:

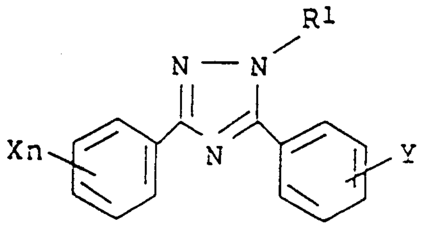

[I]

(wobei $R^1$, X, Y und n die gleiche Bedeutung wie im Anspruch 1 haben,) welches die Reaktion einer Verbindung aufweist, die durch die folgende allgemeine Formel [VIII] dargestellt wird:

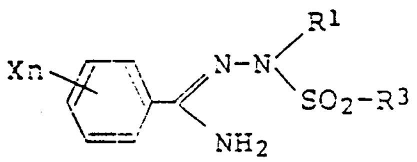

[VIII]

(wobei $R^1$, X, Y und n die gleiche Bedeutung wie oben aufgeführt haben, und $R^3$ Benzol oder Benzol, substituiert mit einer Alkylgruppe mit einer Kohlenstoffzahl von 1-4 ist) mit einer Verbindung, welche durch die folgende allgemeine Formel [V] dargestellt wird:

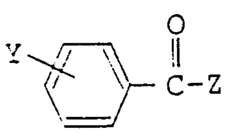

[V]

(worin Z ein Halogenatom ist und Y die gleiche Bedeutung wie oben aufgeführt hat.

**Revendications**

1. Dérivé de triazole ayant la formule générale suivante:

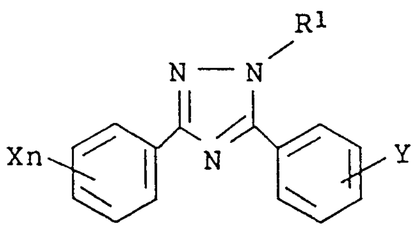

[I]

[dans laquelle $R^1$ est un groupe alkyle, X est un atome d'hydrogène, un atome d'halogène, un groupe alkyle, un groupe alcoxy, un groupe alkylthio, un groupe nitro, un groupe cyano ou un groupe trifluorométhyle, n est un entier de 1 à 5, du moment que, quand n vaut 2 ou plus, X peut être une combinaison quelconque d'atomes ou groupes identiques ou différents, et Y est un groupe alcényle, un groupe alcynyle, un groupe alcoxyalkyle, un groupe alcoxyalcoxy, un groupe alkylthioalkyle, un groupe cyclo-alkyle, un groupe cycloalkylakcoxy, un groupe cycloalkylalkyle, un groupe cycloalkylalcényle, un groupe cycloalkylalcynyle, un groupe trialkylsilylalkyle, un groupe trialkylsilylalcoxy, un groupe alkyle n'ayant pas moins de 7 atomes de carbone, un groupe alcoxy n'ayant pas moins de 7 atomes de carbone, un groupe alkylthio n'ayant pas moins de 7 atomes de carbone, un groupe alkylsulfynyle n'ayant pas moins de 7 atomes de carbone, un groupe alkylsulfonyle n'ayant pas moins de 7 atomes de carbone

ou un groupe représenté par la formule générale (1):

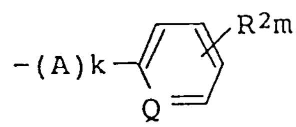

(dans laquelle A est un atome d'oxygène, un atome de soufre, un groupe alkylène inférieur en $C_1$ à $C_4$, un groupe alkylène-oxy inférieur en $C_1$ à $C_4$, un groupe oxy(alkylène inférieur en $C_1$ à $C_4$) ou un groupe alkylène-oxyalkylène inférieur en $C_2$ à $C_8$, k vaut 0 ou 1, Q est un groupe CH-ou un atome d'azote, $R^2$ est un atome d'hydrogène, un atome d'halogène, un groupe alkyle, un groupe alcoxy, le groupe trifluorométhyle ou le groupe trifluorométhoxy, m est un entier de 1 à 5, du moment que, quand m vaut 2 ou plus, $R^2$ peut être une combinaison quelconque d'atomes ou groupes identiques ou différents)].

2. Dérivé de triazole selon la revendication 1, dans lequel ledit radical $R^1$ est un groupe alkyle à chaîne droite ou ramifiée, ayant de 1 à 6 atomes de carbone, X est un atome d'hydrogène, un atome d'halogène, un groupe alkyle à chaîne droite ou ramifiée ayant de 1 à 4 atomes de carbone, un groupe nitro, un groupe cyano ou un groupe trifluorométhyle, n est un entier de 1 à 3, du moment que, quand n vaut 2 ou 3, X peut être une combinaison quelconque d'atomes ou groupes identiques ou différents, Y est un groupe alkyle à chaîne droite ou ramifiée, ayant de 7 à 20 atomes de carbone, un groupe cycloalkyle ayant de 3 à 12 atomes de carbone, un groupe cycloalkylalkyle ayant de 6 à 12 atomes de carbone, un groupe alcoxy à chaîne droite ou ramifiée ayant de 7 à 16 atomes de carbone, un groupe cycloalkylalcoxy ayant de 7 à 12 atomes de carbone, un groupe alkylthio à chaîne droite ou ramifiée ayant de 7 à 16 atomes de carbone, un groupe alkylsulfynyle, un groupe alkylsulfonyle, un groupe alcényle à chaîne droite ou ramifiée ayant de 3 à 16 atomes de carbone, un groupe cycloalkylalcényle ayant de 5 à 12 atomes de carbone, un groupe alcynyle à chaîne droite ou ramifiée ayant de 3 à 16 atomes de carbone, un groupe cycloalkylalcynyle ayant de 5 à 12 atomes de carbone, un groupe tri(alkyle inférieur en $C_1$ à $C_6$) silyl(alkyle inférieur en $C_1$ à $C_6$), un groupe tri(alkyle inférieur en $C_1$ à $C_6$)silyl(alcoxy inférieur en $C_1$ à $C_6$) ou un groupe représenté par ladite formule (1) dans laquelle A est un atome d'oxygène, un atome de soufre, un groupe alkylène inférieur ayant de 1 à 4 atomes de carbone, le groupe méthylène-oxy ou le groupe oxyméthylène, k vaut 0 ou 1, Q est un groupe CH- ou un atome d'azote, $R^2$ est un atome d'hydrogène, un atome d'halogène, un groupe alkyle inférieur en $C_1$ à $C_6$, un groupe alcoxy inférieur en $C_1$ à $C_6$, le groupe trifluorométhyle ou le groupe trifluorométhoxy, et m est un entier de 1 à 3, du moment que, quand m vaut 2 ou 3, $R^2$ peut être une combinaison quelconque d'atomes ou groupes identiques ou différents).

3. Dérivé de triazole selon la revendication 2, dans lequel ledit radical $R^1$ est le groupe méthyle.

4. Insecticide contenant comme matière active un dérivé du triazole selon la revendication 1.

5. Acaracide contenant comme matière active un dérivé du triazole selon la revendication 1.

6. Procédé de production d'un dérivé du triazole ayant la formule générale (I) :

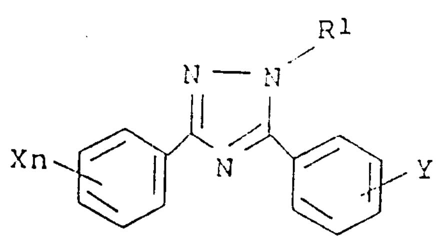

[ I ]

(dans laquelle $R^1$, X, Y et n ont les mêmes significations que dans la revendication 1), qui consiste à faire réagir un composé représenté par la formule générale (II) ci-après :

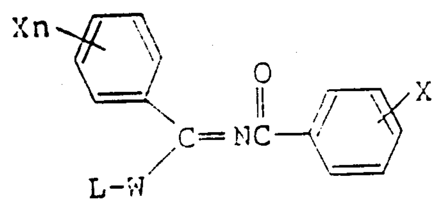

[ II ]

(dans laquelle W est un atome de soufre ou un atome d'oxygène, L est un groupe alkyle ayant de 1 à 4 atomes de carbone et X, n et Y ont les mêmes significations que ci-dessus) avec un dérivé d'une hydrazine représentée par la formule générale (III) $R^1NHNH_2$ (où $R^1$ a les mêmes significations que ci-dessus).

**7.** Procédé de production d'un dérivé du triazole ayant la formule générale (I) ci-après :

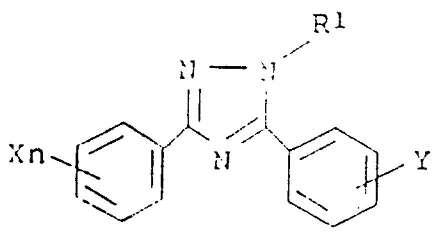

[ I ]

(dans laquelle $R^1$, X, Y et n ont les significations données dans la revendication 1), qui consiste à faire réagir un composé représenté par la formule générale (VI) ci-après :

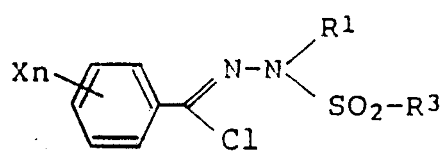

[ VI ]

(dans laquelle $R^1$, X, n et Y ont les mêmes significations que ci-dessus, et $R^3$ est le benzène, ou un benzène substitué par un groupe alkyle ayant de 1 à 4 atomes de carbone) avec un dérivé du benzonitrile représenté par la formule générale (VII) ci-après :

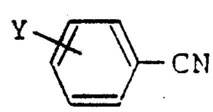

[ VII ] ·

(dans laquelle Y a les mêmes significations que ci-dessus) en présence d'un acide de Lewis.

**8.** Procédé de production d'un dérivé du triazole ayant la formule générale (I) ci-après :

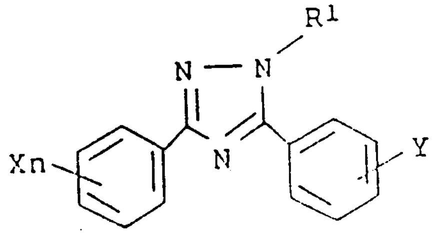

[ I ]

(dans laquelle $R^1$, X, Y et n ont les mêmes significations que dans la revendication 1), qui consiste à faire réagir un composé représenté par la formule générale (VIII) ci-après :

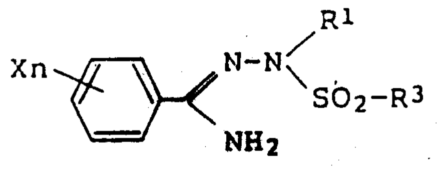

[ VIII ]

(dans laquelle $R^1$, X, n ont les mêmes significations que ci-dessus, et $R^3$ est le benzène, ou un benzène substitué par un groupe alkyle ayant de 1 à 4 atomes de carbone) avec un composé représenté par la formule générale (V) ci-après :

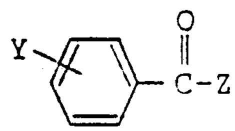

[ V ]

(dans laquelle Z est un atome d'halogène et Y a les mêmes significations que ci-dessus).